# EUROPEAN PATENT SPECIFICATION

(11) **EP 2 522 675 B1**
(45) Date of publication and mention of the grant of the patent: **30.11.2016**
(21) Application number: 11166057.7
(22) Date of filing: 13.05.2011
(51) Int. Cl.: C07K 14/415, C12N 15/82, C12N 5/10, A01H 5/00

(54) **SpBRANCHED1a of Solanum pennellii and tomato plants with reduced branching comprising this heterologous SpBRANCHED1a gene**
SpBRANCHED1a von Solanum pennellii und Tomatenpflanzen mit reduzierter Verzweigung mit diesem heterologen SpBRANCHED1a-Gen
SpBRANCHED1a de Solanum pennellii dans des plants de tomate avec une ramification réduite comprenant ce gène SpBRANCHED1a hétérologue

(43) Date of publication of application: 14.11.2012
(73) Proprietor: Consejo Superior De Investigaciones Científicas (CSIC), 28006 Madrid (ES)
(72) Inventor: Gonzalez-Grandio, Eduardo, 28049 Madrid (ES); Cubas Dominguez, Pilar, 28049 Madrid (ES)
(74) Representative: Pons

(56) References cited:
- WO-A1-2010/081917
- ESHED YUVAL ET AL: "An introgression line population of Lycopersicon pennellii in the cultivated tomato enables the identification and fine mapping of yield-associated QTL", GENETICS, GENETICS SOCIETY OF AMERICA, AUSTIN, TX, US, vol. 141, no. 3, 1 January 1995 (1995-01-01), pages 1147-1162, XP002573291, ISSN: 0016-6731 & C.M. RICK: "Second generation Solanum pennellii (syn. L. pennellii) introgression lines", TGRC - TOMATO GENETICS RESOURCE CENTER, 15 April 2002 (2002-04-15), Retrieved from the Internet: URL:http://tgrc.ucdavis.edu/pennellii_ils. aspx> [retrieved on 2011-09-14]
- AGUILAR-MARTINEZ JOSE ANTONIO ET AL: "Arabidopsis BRANCHED1 acts as an integrator of branching signals within axillary buds", PLANT CELL, vol. 19, no. 2, February 2007 (2007-02), pages 458-472, XP000002659265, ISSN: 1040-4651
- CUBAS P ET AL: "The TCP domain: a motif found in proteins regulating plant growth and development", PLANT JOURNAL, BLACKWELL SCIENTIFIC PUBLICATIONS, OXFORD, GB, vol. 18, no. 2, 1 January 1999 (1999-01-01), pages 215-222, XP002419545, ISSN: 0960-7412, DOI: 10.1046/J.1365-313X.1999.00444.X
- SCHMITZ G ET AL: "Genetic control of branching in Arabidopsis and tomato.", CURRENT OPINION IN PLANT BIOLOGY FEB 1999 LNKD- PUBMED:10047573, vol. 2, no. 1, February 1999 (1999-02), pages 51-55, XP000002659266, ISSN: 1369-5266
- MARTIN-TRILLO MAR ET AL: "Role of tomato BRANCHED1-like genes in the control of shoot branching", PLANT JOURNAL, vol. 67, no. 4, 21 June 2011 (2011-06-21), pages 701-714, XP000002659267,

## Description

The present invention falls within the section of human necessities, in the field of agriculture; new plants or processes for obtaining them by means of biotechnology; flowering angiosperms plants, and specifically tomato plants. In particular, the present invention relates to the *SpBRANCHED1a* gene of *Solanum pennellii,* and tomato plants comprising it heterologously, wherein these tomato plants have fewer and/or shorter axillary shoots than a control plant.

### BACKGROUND ART

A central question in Biology is how the molecular evolution of genomes has led to the great variety of morphologies found in living organisms. Duplication and divergence of developmental genes is thought to have played a key role in the emergence of new traits. Genome-wide phylogenetic studies are helping us elucidate the evolutionary history of duplications in some of these gene families. However, the molecular mechanisms underlying functional divergence following duplication, and the relationship between gene evolution and emergence of morphological novelties are still not well understood.

In angiosperms, branching patterns greatly determine overall plant architecture and affect key aspects of plant life such as nutrient allocation, height, light harvesting efficiency and visibility for pollinators. Branches are generated from meristems formed in the axils of leaves (axillary meristems, AMs) after germination. AMs develop into *axillary buds,* structures comprising short internodes and nodes bearing leaf primordia and further AMs. Buds may remain dormant for long periods of time or undergo internode elongation to generate lateral shoots. Recent studies suggest that axillary bud development is controlled by a conserved genetic network evolved before the radiation of flowering plants. AM initiation is controlled by Lateral suppressor/*MONOCULM1* genes, Blind/*RAX1* genes, and *LAX PANICLE* genes. Auxin and strigolactone, hormones synthesised in the shoot apex and the root respectively, act as long distance signals to suppress branching. Synthesis and response to strigolactone is controlled by the More axillary branches/Ramosus pathway (MAX/RMS). Genes responding to these signals and acting inside the bud to prevent outgrowth are also conserved: maize *teosinte branched1* (*tb1*) and its orthologs promote bud arrest in monocots (Doebley et al. 1997. Nature, 386: 485-488; Hubbard et al. 2002. Genetics, 162: 1927-1935; Hu et al. 2003. J. Plant Physiol. Mol. Biol., 29: 507-514; Kebrom et al. 2006. Plant Physiol, 140: 1109-1117; Takeda et al. 2003. Plant J., 33: 513-520). In dicots, duplications of an ancestral tb1-like gene generated the *CYC*/*TB1* clade comprising three types of genes (Howarth and Donoghue 2006. J. Plant Physiol. Mol. Biol., 29: 507-514). One of them includes *BRANCHED1* (*BRC1*) which has retained most of the branch-suppressing activity in Arabidopsis (Aguilar-Martinez et al. 2007. Plant Cell, 19: 458-472). *tb1*/*BRC1* genes encode transcription factors of the TCP family (Cubas et al. 1999. Plant J., 18: 215-222). TCP transcription factors are involved in the control of cell growth and proliferation in meristems and lateral organs, and they have played a key role in the evolution of innovations such as flower zygomorphy.

Despite the general conservation of genes and pathways, a great diversity of branching patterns is found in angiosperms. Moreover, timing of AM initiation, and branch outgrowth in response to environmental and endogenous signals are very divergent among clades. Molecular evolution of genes controlling branch outgrowth must have played an important role in the generation of this diversity. Putative candidate genes to be targets for selection during the evolution of new branching patterns are *tb1*/*BRC1-like* genes. Indeed, in maize, artificial selection of *tb1* over-expressing alleles was responsible for the strong apical dominance of the domesticated maize plants (Doebley, Stec and Hubbard 1997, Wang et al. 1999. Nature, 386: 485-488).

*BRC1*-like genes from *Solanum lycopersicum* (Solanaceae, Asteridae), a dicot species distantly related to Arabidopsis (Brassicaceae, Rosidae) and with diverging branching patterns. In contrast to Arabidopsis, which has an indeterminate (monopodial) growth habit, tomato, has a determinate (sympodial) growth habit: while in Arabidopsis the shoot apical meristem (SAM) grows indefinitely, in tomato, after the production of 8-12 leaves, the SAM is terminated in a cymose inflorescence. Upon flowering, the lateral meristem in the uppermost leaf axil (sympodial bud) is immediately released to give a shoot. This lateral shoot generates several leaves before terminating with another inflorescence, and so on. Additional lateral shoots may grow out from buds of the primary shoot.

In the international patent application WO 2010/081917 A1 was described the sequence of *SIBRC1* from *Solanum lycopersicum* and inventors elucidated, by means of RNAi experiments, that the *SIBRC1b* gene plays a more important role in the control of axillary bud development and branch outgrowth than *SIBRC1a.* Furthermore, this patent application related to the promoter of said gene. Despite the results shown in that document, it remains in the state of the art the problem to produce plants that have effectively a substantial reduction in the number of branches in order obtain plants with better fruits and also to improve the mechanical harvesting of these fruits.

### SUMMARY OF THE INVENTION

The invention relates to a new sequence of *BRANCHED1a* gene, the *SpBRANCHED1a* gene of *Solanum pennellii,* and to an expression product of said gene, to a vector, host cell, cell culture, recombinant expression system, or tomato plants comprising said vector. The present invention also refers to the heterologous use of the gene in tomato plants, to produce a plant having fewer and/or shorter axillary shoots than a control plant, and to a method for producing tomato plants having fewer and/or shorter axillary shoots than a control plant.

In the present disclosure the term *SpBRANCHED1a* as synonymous of *SpBRC1a* can be used. Along the present disclosure to refer to this gene can be used the term "isolated polynucleotide" or "polynucleotide" encoding a polypeptide comprising the amino acidic sequence of the protein *SpBRANCHED1a* (*SpBRC1a*) from *Solanum pennellii,* taking into account that, by alternative splicing, two proteins are translated from a single genomic sequence, a short and a long protein, differing in their C-terminal domain.

As it is known in the state of the art, in the case of tomato *BRC1*-like genes, *SIBRC1b* seems to have retained the ancestral *BRC1*-like gene function in the suppression of shoot branching while inventors have not been able to establish any relevant role in this process for *SIBRC1a.* Consistently, an asymmetrical distribution of selective constraints has been detected in the evolution of *Solanum BRC1a* and *BRC1b* lineages: the coding sequence of *BRC1b* genes have evolved under a strong purifying selection while the *BRC1a* lineage had experienced a decrease of evolutionary constraints. Therefore, despite this evidence, the present invention shows that *SpBRC1a* plays an unexpected role in suppressing shoot branching when is expressed heterologously in tomato, in opposition with the non-contributing role to this branchless phenotype of the native gen *SIBRC1a.*

The distinguishing features of the present invention in respect to the relevant background art is the new sequence *SpBRC1a* from *Solanum pennellii* and its use to produce plants which express *SpBRC1a* heterologously in tomato plants, as for example, in *Solanum lycopersicum* plants. The technical effect resulting from the distinguishing features is a stronger apical dominance, an improved architecture and more reduced number of lateral branches (branchless phenotype) than control plants, M82 tomato plants. This technical effect implies an improvement of the described phenotype with respect to control plants, described in the examples of the present invention (e.g. IL3-5 plants). This improvement is due to the functional substitution of the native gene *SIBRC1a* from *Solanum lycopersicum.* Therefore, the expression of *SpBRC1a* in *Solanum lycopersicum* implies an improvement of branchless phenotype in tomato plants.

Then, the objective technical problem solved by the present invention is the production of tomato plants with the maximal reduction in number and length of axillary shoots as possible because lateral shoot branching is an undesired trait in the domesticated tomato as it diverts assimilates away from developing fruits.

In conclusion, the present invention provides new plants with branchless phenotype modifying or adapting the closest prior art to provide the described technical effect. Therefore, the present invention provides tools to the state of the art to improve the branchless phenotype in tomato plants.

It must be stressed that the present invention emphasizes an unexpected or surprising effect: Since is not *SIBRC1a* but *SIBRC1b* the sole responsible in controlling the shoot branching in *Solanum lycopersicum* plants, the native *SIBRCla* gene does not play a major role in the suppression of axillary bud outgrowth in these tomato plants. In the state of the art is not suggested that any homologous gene of *SIBRC1a* in any tomato plant would have a different activity/function than it is demonstrate with *SIBRC1a,* despite the high identity between proteins SpBRC1a and SIBRC1a, being 92-93% identical (table 1), the role of SpBRC1 is very different when expressed in *Solanum Lycopersicum* than the role of the native protein SIBRC1a.

The plants of the present invention also have the advantage to facilitate the pruning and more important than this, because the plants of the invention don't need the pruning of lateral shoots, fewer wounds can be practice and then, potentially, a less number of pathogens will infect the plant.

The present invention is susceptible of industrial application, i.e. for avoid the deviation of assimilates away from developing fruits and also to improve the mechanical harvesting of tomatoes.

Thus, a first aspect of the present invention refers to an isolated polynucleotide, encoding a polypeptide comprising:
a. an amino acidic sequence from *Solanum pennellii* which is at least 95% identical over the full length of SEQ ID NO:1 or of SEQ ID NO: 2, or
b. the amino acidic sequence from *Solanum pennellii* SEQ ID NO: 1 or SEQ ID NO: 2.

In the present invention, the terms "polypeptide" and "amino acidic sequence" are synonymous.

The amino acidic sequence from *Solanum pennellii* which is at least 90% identical to SEQ ID NO: 1 or which is at least 90% identical to SEQ ID NO: 2 are isoforms of these sequences or sequences with a maximum of 10% of changes in amino acids between them sequences having substantially the same activity/role in the branchless phenotype than SEQ ID NO: 1 or SEQ ID NO: 2. Therefore, the present disclosure also refers to an isolated polynucleotide, encoding a polypeptide comprising:
a. the amino acidic sequence of any protein SpBRC1 a from *Solanum pennellii,* wherein the protein SpBRC1a has substantially the same role in the branchless phenotype than SEQ ID NO: 1 and/or SEQ ID NO: 2 in tomato plants, or
b. the amino acidic sequence from *Solanum pennellii* SEQ ID NO: 1 or SEQ ID NO: 2.

SEQ ID NO: 1 or SEQ ID NO: 2 are the two resulting sequences of alternative splicing of the gene *SpBRC1a,* coded in the genomic sequence (example 1). Since the putative splicing sites (gt/ag) of intron I were found in all the *Solanum BRC1a* genes studied but not in *BRC1b* genes, inventors demonstrate that this pattern of alternative splicing of *BRC1a* gene was maintained in respect to *SpBRC1a* gene identifying said intron I in the genomic sequence of *SpBRC1a* gene. Then, these two sequences SEQ ID NO: 1 and SEQ ID NO: 2 are involved in the branchless phenotype. The two sequences have different C-terminal (C-t) domains due to a frameshift caused by the alternative splicing of intron I, like in *SIBRC1a* gene.

A preferred embodiment refers to the isolated polynucleotide wherein said polynucleotide is SEQ ID NO: 3 encodes SEQ ID NO: 1, or SEQ ID NO: 4 encodes SEQ ID NO: 2. The sequence SEQ ID NO: 3 is the Coding Sequence (CDS) of the short variant of SpBRC1a protein; SEQ ID NO: 1. The sequence SEQ ID NO: 4 is the CDS of the long variant of SpBRC1a protein; SEQ ID NO: 2.

In another preferred embodiment of the present invention, the isolated polynucleotide is the genomic sequence SEQ ID NO: 5. This sequence has the introns described for *SIBRC1a.*

The term "% identical" as is understood in this disclosure refers to the % of identity between two amino acid sequences. The % of identity is a count of the number of positions over the length of the alignment of two sequences where all of the amino acids at that position are identical.

Preferably, the amino acidic sequence from *Solanum pennellii* which is at least 90% identical to SEQ ID NO: 1 or to SEQ ID NO: 2 can be at least 91%, 92%, 93%, 94%, 95%, 96%, 97%, 98% or 99% identical to SEQ ID NO: 1 or to SEQ ID NO: 2. Furthermore, the amino acidic sequence from *Solanum pennellii* which is at least 90% identical to SEQ ID NO: 1 or which is at least 90% identical to SEQ ID NO: 2 can be an amino acidic sequence with at least less than 90% identical to SEQ ID NO: 1 or SEQ ID NO: 2 if said amino acidic sequence has substantially the same role in the branchless phenotype of SEQ ID NO: 1 and/or SEQ ID NO: 2 in tomato plants, and also is a sequence from *Solanum pennellii.*

In order to yield information about of the identity of different sequences of BRC1 a proteins in tomato plants, table 1 and table 2 is presented.

**Table 1. Percentage of identity of several homologous amino acidic sequences respect to SEQ ID NO: 1 or SEQ ID NO: 2 from different tomato plants.**

| **Species 1** | **Size (amino acids)** | **Species 2** | **Size (amino acids)** | **Identity (%)** |
|---|---|---|---|---|
| S. *pennellii* Short (SEQ ID NO: 1) | 313 | *S. lycopersicum* Short (SEQ ID NO: 8) | 325 | 92 |
| | | S. *tuberosum* Short (SEQ ID NO: 9) | 300 | 85 |
| S. *pennellii* Long (SEQ ID NO: 2) | 349 | S. *lycopersicum* Long (SEQ ID NO: 10) | 346 | 93 |
| | | S. *tuberosum* Long (SEQ ID NO: 11) | 336 | 86 |

The methods to compare the sequences obtaining identity percentages are known in the state of the art, and include, but not limited to, the program GAG, including GAP (Devereux *et al.* 1984. Nucleic Acids Research, 12: 287), Genetics Computer Group University of Wisconsin, Madison, (WI); BLAST, BLASTP, ClustallW2 or Phylemon web server (Tarraga *et al.* 2007. Nucleic Acids Res, 35: W38-42). The percentage of identity has been chosen according to the ClustallW2 Multiple Sequence Alignment from European Bioinformatics Institute.

As it is shown in table 1, despite the high identity between proteins SpBRC1a and SpBRC1a, being 92-93% identical, the role of SpBRC1a very different, as demonstrated in the present invention, when expressed in *Solanum Lycopersicum* compared with the role of the native protein SIBRC1a.

**Table 2. Percentage of identity between SEQ ID NO: 1, SEQ ID NO: 2 (SpBRC1a) and SpBRC1b from Solanum pennellii tomato plants.**

| **Species 1** | **Size (amino acids**) | **Species 2** | **Size (amino acids**) | **Identity (%)** |
|---|---|---|---|---|
| *S. pennellii* Short (SEQ ID NO: 1) | 313 | *S. pennellii* Long (SEQ ID NO: 2) | 349 | 92 |
| S. *pennellii* Short (SEQ ID NO: 1) | 313 | S. *pennellii* SpBRC1b (SEQ ID NO: 10) | 362 | 37 |
| S. *pennellii* Long (SEQ ID NO: 2) | 349 | S. *pennellii* SpBRC1b (SEQ ID NO: 10) | 362 | 42 |

A preferred embodiment of the present invention refers to the polynucleotide operatively linked to any regulatory sequence that controls the expression of said polynucleotide. In further preferred embodiment of the present invention, said regulatory sequence is SEQ ID NO: 6, SEQ ID NO: 7 or CaMV35S.

The term "regulatory sequence that controls the expression of a polynucleotide" refers to a nucleotidic sequence having an effect on the functionality of the polynucleotide gene referred to the beginning of the transcript from a DNA sequence or at the start of translation of a sequence of RNA or other sequences not described. By way of example, between regulatory sequences of the gene expression referred to in the present disclosure are the promoters and other less common as certain introns. The nature of these regulatory sequences differs depending on the host organism; in prokaryotes, these sequences of control usually include a promoter, a site of union ribosomal, and signs of completion; in eukaryotes, usually, these sequences of control include promoters, signs of termination, intensifiers and sometimes silencers.

As it is used here, the term "promoter" refers to a region of the DNA upstream from the start point of the transcription of a gene, and particularly, that is able to start the transcription in a plant cell. Examples of promoters include but are not limited to, promoters obtained from plants, virus of plants or bacteria that can express genes in cells of plants, as *Agrobacterium* or *Rhizobium.* Promoters can be classified, for instance, as inducible and constitutive promoters. The sequence SEQ ID NO: 6 is the inducible promoter of the gene *SIBRC1a* from *Solanum lycopersicum.* The sequence SEQ ID NO: 7 is the inducible promoter of the gene *SIBRC1a* from *Solanum tuberosum.*

The term "operatively linked" refers to a juxtaposition in which the components as well described have a relationship that enables them to work in the intentional way. A polynucleotide operatively linked to any regulatory sequence that controls the expression of said polynucleotide is linked in such a way that the expression of the coding sequence of the polynucleotide is achieved under conditions compatibles with the regulatory sequence.

Another aspect of the present invention refers to an expression product of the polynucleotide of the invention. To refer to any of the expression product of the polynucleotide of the present invention, the term "expression product of the invention" or "expression product of the present invention" can be used.

The term "product of the expression of the sequence" as it is used in the present disclosure refers to any product derived from the expression of the polynucleotide of the disclosure. Thus, as product derived from the expression of the polynucleotide of the disclosure is understood, for example, the RNA that is obtained from the transcription of said polynucleotide, the processed RNA, the resulting protein in the translation of RNA, or subsequent modifications to the polynucleotide inside the cell, provided that the resulting sequence of these modified polynucleotides having its origin in the original sequence or the functional role described in the present disclosure.

A further aspect of the present invention refers to an expression vector comprising the polynucleotide of the invention. To refer to any vector comprising the polynucleotide of the present invention, the term "vector of the invention" or "vector of the present invention" can be used.

The term "vector" refers to a DNA fragment that has the ability to replicate in a given host and, as the term indicates, it can serve as a vehicle to multiply another fragment of DNA that has being linked to the same (insert). The term "insert" refers to a DNA fragment that is linked to the vector; in the case of the present invention, the vector comprises any of the polynucleotides of the invention that can be replicated in the appropriate host. The vectors may be plasmids, cosmids, bacteriophages or viral vectors, without excluding another type of vectors that correspond to this definition of vector.

Another aspect of the present invention refers to a host cell comprising the polynucleotide, the expression product, or the vector of the invention, wherein if the host cell is a plant cell, said plant cell is not from the species *Solanum pennellii.* That is to say, the polynucleotide of the present invention (the mean core corresponding to the gen of *Solanum pennelii*) is present in said host cell in heterologous form. A preferred embodiment refers to the host cell of the present invention, wherein said cell is a plant cell. More preferably the plant cell is a *Solanum lycopersicum* cell.

The term "host cell" as it is used in the present invention relates to a eukaryotic plant cell and within this group, more preferably, those cells belonging to the Kingdom Plantae, wherein any of theses cells comprises polynucleotide, the expression product, or the vector of the invention. Thus, the term plant cell comprises at least one cell of the parenchyma, meristematic cell or of any kind of plant cell, differentiated or undifferentiated. Preferably the plant cell is a tomato plant cell and more preferably is a *Solanum lycopersicum* cell.

To refer to any host cell comprising the polynucleotide of this invention, the term "host cell of the invention" or "host cell of the present invention" can be used.

The present disclosure further relates to a cell culture comprising the host cell of the invention. To refer to any cell culture comprising the host cell of the present invention, the term "cell culture of the invention" or "cell culture of the present invention" can be used.
The term "cell culture" refers to a cultivation of cells isolated from the same or different type of tissue, or a collection of these cells organized in parts of a plant or in tissues (tissue culture). Types of this kind of cultures are, e.g. but without any limitation, a culture of protoplasts, calli (groups of plant cells undifferentiated able to regenerate a complete plant with the appropriate organogenic program) or a culture of plant cells that are isolated from plants or parts of plants such as embryos, meristematic cells, pollen, leaves or anthers.

Also disclosed is a recombinant expression system comprising the polynucleotide, the expression product, the vector, or the host cell of the invention.

The recombinant expression system can be, but without limitation, e.g. a recombinant host cell or a recombinant bacteriophage or their combination with any helper virus. Recombinant manufacturing involves the expression of a DNA construct encoding for the desired protein in a recombinant host cell. The host cell can be either prokaryotic or eukaryotic. Recombinant manufacturing, however, does have its difficulties. Expression constructs must be optimized for a particular protein and for a particular host cell. Expressing a recombinant protein in a host cell exposes the recombinant protein to a new set of host cell enzymes, such as proteases, which can modify or even degrade the recombinant protein. Modification and degradation of the recombinant protein of interest is undesirable.

A further aspect of the present invention refers to a use of the expression vector or the host cell of the invention, to produce a tomato plant, but not a plant of the species *Solanum pennellii,* having fewer and/or shorter axillary shoots than a control plant.

The term "axillary shoots" can be used as synonymous of "lateral branches", "lateral shoots", "secondary shoots", "axillary shoot branching", "axillary bud outgrowth", and refers to a shoot growing from the axillary bud located between the leaf and the stem, that is to say, growing in a node. In a control plant, the stem displays branching due the outgrowth of axillary buds in the individual node positions.

In the tomato plants of the present disclosure the length of the basal lateral branches (nodes 1-6) presents significant differences with regard to the length of the lateral branches in the control.

In the present disclosure the term "fewer and/or shorter" relates to significant differences between the presence/absence and/or the length of axillary shoots, respectively, in the equivalent node of the plants of the disclosure and control plants. The skilled person can arrive to these significant differences using any statistical and any method known in the background art. Then, in the present disclosure platns having fewer and/or shorter axillary shoots than a control plant have branchless phenotype.

For instance, as shown in the examples of the present invention, IL3-5 plants had significantly fewer and/or shorter axillary shoots than M82 plants in cotyledons, and nodes 1, 4, 5 and 6.

The control plant is any tomato plant, wild type carrying empty vectors, but in any case the control plant does not contain the polynucleotide of the present invention. Preferably the control plant is a tomato plant belonging to the species *Solanum lycopersicum,* preferably the control plant is a M82 cultivar of tomato. The control plant has the same taxonomic category than the tomato plant comprising the polynucleotide of the invention in order to compare the number or/and length of lateral branches.

Another aspect of the present disclosure refers to a tomato plant, but not a *Solanum pennellii* plant, comprising the polynucleotide, the expression product, the vector, the host cell, the cell culture, or the recombinant expression system of the disclosure, wherein said plant has fewer and/or shorter axillary shoots than a control plant. Thus, the polynucleotide of the present disclosure (the mean core corresponding to the gen of *Solanum pennelii*) is present in plant in heterologous form. Preferably the tomato plant belongs to the species *Solanum lycopersicum.*

The tomato plant of the present disclosure can be selected from potencially tomato plant species, for instance the tomato plant can be selected from tomato plants with red, yellow, orange or yellow-green fruit, and without limitation to theses species: *Solanum lycopersicum, Solanum peruvianum, Solanum pimpinellifolium, Solanum lycopersicoides, Solanum sitiens, Solanum juglandifolium, Solanum ochranthum, Solanum cheesmaniae or Solanum galapagense.*

Preferably, the tomato plant belongs to the species *Solanum lycopersicum.* More preferably the variety of the tomato plant can be selected from the list of stock names in the SOL Genomics network (SGN) Breeders Toolbox (http://solgenomics.net/breeders/index.pl). Preferably the variety of the tomato plant from the species *Solanum lycopersicum* can be selected from the list, but without limitation, Anna russian, Applause, Aussie, Baladre, Bella rosa, Black cherry, Black russian, Blondkopfchen, Brandywine, Carbon, Ceylan, Cherokee purple, Cherry yellow pear, Black Plum, Comanche, Copy, Costoluto genovese, Ditmarcher, Eros, Gallician, Glacier, Gartenperle, Green sausage, Grushovka, Harzfeuer, Hugh, Japanesse black, Jersey devil, Kosovo, Krim black, Kumato, Liguria, Limachino, Lime green salad, Manitoba, Marvel stripe, Moneymaker, Muchamiel, Opalka, RAF, Black Pear, Hawaian pineapple, Rio grande, San marzano, Siberian, Sprite, Sugary, Sun sugar, Tigerella, Valencian, White Queen or Window box Roma.

A further aspect of the present disclosure refers to a tomato plant comprising, heterologously, a fragment of the chromosome III of *Solanum pennellii* that comprises a polynucleotide encoding a polypeptide comprising:
a. an amino acidic sequence from *Solanum pennellii* which is at least 90% identical to SEQ ID NO: 1 or which is at least 90% identical to SEQ ID NO: 2, or b. the amino acidic sequence from *Solanum pennellii* SEQ ID NO: 1 or SEQ ID NO: 2.
, wherein said plant has fewer and/or shorter axillary shoots than a control plant

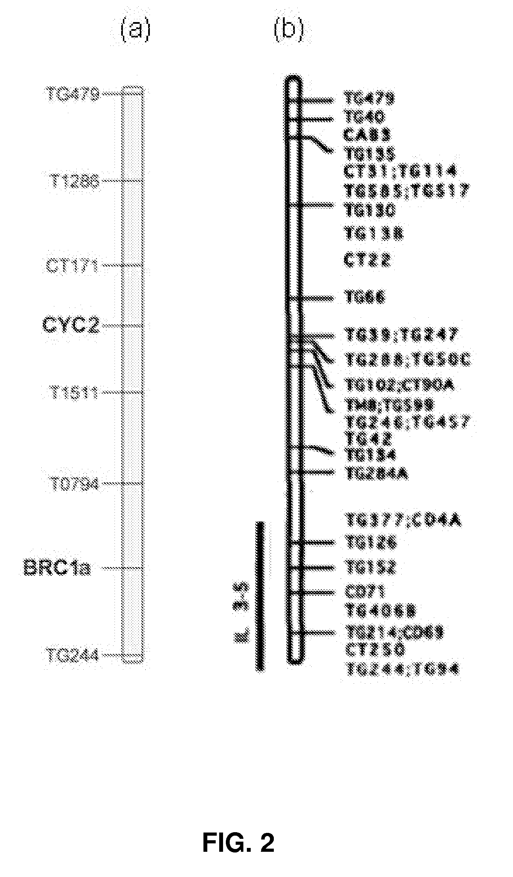

A preferred disclosure refers to said tomato plant comprising, heterologously, a fragment of the chromosome III of *Solanum pennellii* that comprises a polynucleotide encoding a polypeptide, wherein said fragment of the chromosome III corresponds to the fragment comprised between the DNA marker TG284A and the DNA marker TG244 (figure 2). Preferably the fragment of the chromosome III corresponds to the fragment comprised between the DNA marker T0794 and the DNA marker TG244; or more preferably to the fragment comprised between the DNA marker TG377 (or the marker TG126 or CD4A) and the DNA marker TG244. The figure 2 shows all the proposed DNA markers within chromosome III of *Solanum pennellii.* The cited markers can be analyzed in detail in the Sol Genomics Network (SGN) database.

A preferred disclosure refers to the plant comprising, heterologously, a fragment of the chromosome III of *Solanum pennellii,* wherein comprises a polynucleotide encoding a polypeptide comprising SEQ ID NO: 3 encoding SEQ ID NO: 1, or SEQ ID NO: 4 encoding SEQ ID NO: 2. Preferably said polynucleotide is the genomic sequence SEQ ID NO: 5.

Also disclosed is any plant described above wherein the expression of the polynucleotide of the invention is greater than the expression of the homologous native gene *BRC1a.*

The plant contains the polynucleotide of the invention in homozygosis, heterozygosis or hemicigosis. Also disclosed is any plant described above, wherein the polynucleotide is integrated in their genome, preferably integrated in homozygosis Another aspect refers to a germplasm of the plant of the present invention. Preferably the germplasm is a seed or pollen.

The term "plant of the present invention" includes each of the parties on said plant, which can be conserved or cultivated in isolation or in combination, as well as the germplasm. The germplasm is defined by the biological material that contains the intraspecific genetic variability or by the genetic materials that can perpetuate a species or a population of said plant. Thus germplasm is the seed, tissue culture for any part of the plant or plants established in *ex situ* collections, without excluding any other material in the scope of this definition.

The pollen has high level of interest since the transmission of the genetic and phenotypic characters can be carried out by the pollination of any plant variety compatible with the pollen that is referenced. In this way can be produce a plant which includes the polynucleotide of the invention, after the respective cross and selection, it can be obtained a plant in which the polynucleotide integrates a suitable number of copies in stable condition in order to obtain the same desirable branchless phenotype in the subsequent generations.

To refer to any of the tomato plants of the present invention, the term "plant of the invention" or "plant of the present invention" can be used.

The plant of the invention does not belongs to the species *Solanum pennellii,* then being the polynucleotide of the present invention expressed in different plant species that *Solanum pennellii,* therefore, the polynucleotide is expressed heterologously.

A further aspect of the present invention refers to a method for producing tomato plants having fewer and/or shorter axillary shoots than a control plant, comprising:
a. Transforming at least a tomato plant cell with the expression vector comprising the polynucleotide according to any of the claims and
b. growing the plant cell obtained in the step (a) in a suitable medium to produce at least a plant which expresses the heterologous polynucleotide. Another embodiment of the present invention refers to the method for producing tomato plants having fewer and/or shorter axillary shoots than a control plant, wherein said tomato plant belongs to the species *Solanum lycopersicum.*

The plant disclosed herein can be achieved by genetic transformation mediated by biolistic, *Agrobacterium tumefaciens* or any other technique known by the skilled in the art (e.g. transformation of protoplasts), that will allow the integration of the polynucleotide of the invention in any of the DNA of the plant; genomic, chloroplastic or the polynucleotide of the invention by crossing and selection.

The suitable medium growing the plant cell obtained in the step (a) is a known medium by the skilled in the art as for example The production of a plant as is indicate in step (b) can be performed by means techniques known by the skilled in the art, for instance:
- The cultivation of embryos: Isolation of zygotic embryos promoting their growth as plant in an artificial environment
- Somatic embryogenesis: Production of embryos from somatic tissues, such as microspores or leaves
- Organogenesis: Production of organs such as stems or roots from various tissues oft he plant.

The plant disclosed herein can be obtained according with other microbiologic processes as for example:
- Obtaining cybrids: It is produced a cell with its cytoplasm and the cytoplasm of the other cell and this cell can be grown in a suitable medium to produce a plant which expresses the heterologous polynucleotide.
- Fusion of somatic cells (preferably protoplast fusion): at the cytoplasmic level hybrid plants can be the result of: (a) The sum of the cytoplasm of both parental; (b) The cytoplasm of a single parental; (c) a cytoplasm hybrid result of recombination of the genomes extranuclears of both cells. The fusion of somatic cells can be applied; to overcome the incompatibility in interspecific crosses, or a better utilization of the interspecies variation and extraspecific in interspecific compatible crosses.

### BRIEF DESCRIPTION OF THE FIGURES

In order to complement the description that is being made and in order to help better understanding of the characteristics of the invention and in agreement with some preferred examples, figures have been included where, with illustrative and non-limiting character, the following is represented:

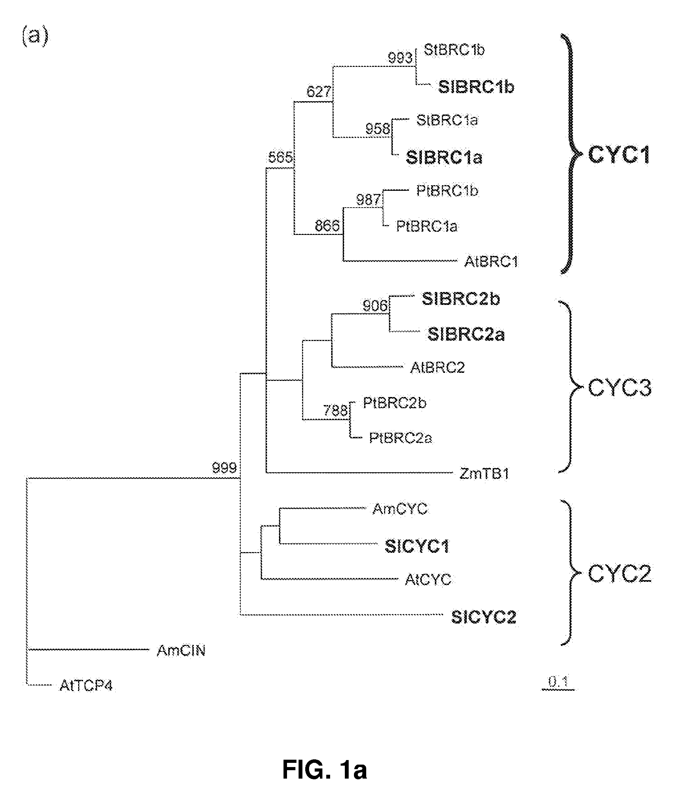

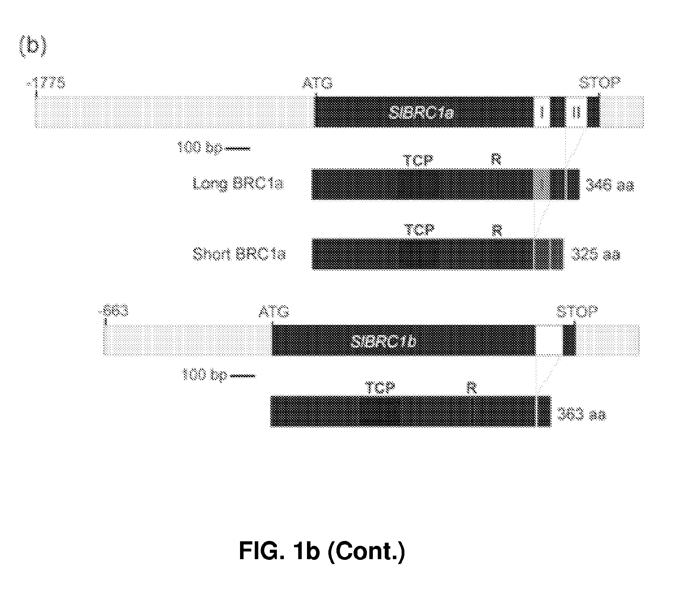

**FIG. 1****. Shows the *BRC1*-like genes and proteins in tomato.**
   (a) Maximum Likelihood (ML) phylogenetic tree with 1000 bootstrap pseudoreplicates of class II TB1/CYC genes from tomato (SI, bold) and representative class II TCP members from *Antirrhinum majus* (Am), *Arabidopsis thaliana* (At), *Solanum tuberosum* (St) and *Populus trichocarpa* (Pt). Branches with support of 500 or more are indicated. AtTCP4/CIN are the outgroups. (b) Gene structure of *SIBRC1a* and *SIBRC1b.* Coding sequences are shaded in black, introns in white, 5' UTR and 3'UTR in light grey. Below each gene, predicted proteins encoded by the isolated cDNAs are represented. Blue boxes indicate TCP and R domains. Red boxes indicate the alternative coding frame in the short SIBRC1a protein. Dark grey box indicates the alternative intron translated in the long protein.
**FIG. 2****. Shows the map of chromosome III of *Solanum lycopersicum* (a) and *Solanum pennellii* (b) and the relative position of *BRC1* gene.**
   In both maps are indicated several DNA markers and their relative position between them and between BCR1 a gene and IL3-5 introgressed fragment.
   TG479, T1286, CT171, T1511, T0794, TG244 are DNA markers from chromosome III of *Solanum lycopersicum* and TG40, CAB3, TG13S, CT31, TG114, TG585, TGS17, TG130, TG13B, CT22, TG66, TG39, TG247, TG288, TG50C, TG102, CT90A, TM8, TGS99, TG246, TG457, TG42, TG134, TG284A, TG377, CD4A, TG126, TG152, CD71, TG406B, TG214, CD69, CT250, TG244 and TG94. The fragment of the Chromosome III from *Solanum Pennelli* introgressed in *Solanum lycopersicum* in IL3-5 introgression line is shown with the bar IL 3-5. (a) and (b) shows some common DNA markers in both chromosomes.
**FIG. 3****. Shows the Secondary structure prediction of the long (top) and short (bottom) C-t motifs of SIBRC1a according to Jpred.**
   E, extended secondary structure, H, α-helix. Jnet, solvent accessibility predictions. B, buried, -, exposed. Jnet Rel, reliability of prediction accuracy, ranges from 0 to 9.
**FIG. 4****. Shows the analysis of *SIBRC1a* function.**
   (a) Shoot branching phenotype of *Slbrca*-v171i point mutants compared to their wild type siblings. Lateral branch length of mutant tomato plants and their wild type siblings at anthesis of the first flower. Standard errors (±s.e.m.) are represented. N*_{brc1a-v171i}*=7, N_{wt}=25. (b) Shoot branching phenotype of IL3-5 and M82 plants measured as in (a) N*_{IL3-5}*=12, N_{wt M82}=15. Asterisks indicate significant differences (p<0.05). (c) Young M82 wild type (left) and IL3-5 (right) plants grown in the same conditions. Arrows indicate branches grown out in wild type plants and arrested buds in IL3-5. (d). Relative *SIBRC1a* mRNA levels in axillary buds of M82 and IL3-5 plants. The asterisk indicates a significant difference (p<0.05).

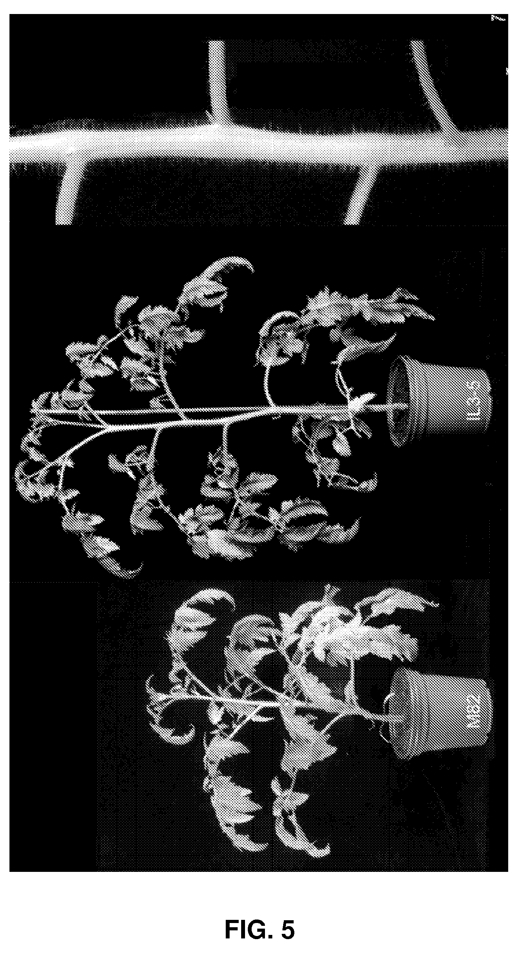

**FIG. 5****. Shows the phenotype of adult M82 and IL3-5 plants after flowering.**

### EXAMPLES

The following examples provide a description, of illustrative and non-limiting character, of some of the assays and operating conditions claimed in the list given below that refers to the cloning and use *SpBRC1a* as a suppressor of shoot branching in tomato, as well as plants of *Solanum lycopersicum* expressing the *SpBRC1a* gen.

In the present examples the term *SpBRC1a* is equivalent to an isolated polynucleotide encoding a polypeptide comprising the amino acidic sequence of SEQ ID NO: 1 (short splicing alternative protein) or SEQ ID NO: 2 (long splicing alternative protein). *SIBRC1a* and *SIBRC1b* refer to the gen *BRANCHED1a* or *BRANCHED1b* of *Solanum lycopersicum.*

### EXAMPLE 1. Identification of tomato BRC1-like genes

The inventors searched for *CYC*/*TB1* type genes in tomato genomic and EST databases (Mueller 2009. The Plant Genome, 2: 78-92) and identified six TCP genes of this group. Phylogenetic analyses showed that two were *CYCLOIDEA* (*CYC*)-like genes, two were *BRC2-*like genes and another two were *BRC1*-like genes. These six TCP genes were named *SICYC1, SICYC2, SIBRC2a, SIBRC2b, SIBRC1a* and *SIBRC1b (Solanum lycopersicum CYC, BRC2, BRC1*) respectively (Figure 1 a) and were mapped to tomato chromosomes 2-6 (Figure 2). *CYC*-like genes have been shown to be expressed in floral meristems and to be mainly involved in the control of flower shape in many species. Inventors focussed on the two *BRC1*-like genes (*SIBRC1a* and *SIBRC1b*) and investigated their roles during tomato lateral shoot development.

Inventors first studied the transcriptional activity of these genes. For *SIBRC1a,* inventors isolated two cDNAs generated by alternative splicing: a short one, with a 978 base pairs (bp) long open reading frame (ORF) split by two introns (introns I and II), and a long one (1041 bp), in which intron I was retained and translated (Figure 1 b). Splicing of intron I was a relatively rare event as long cDNAs were isolated 9 times more frequently than short cDNAs. These cDNAs encoded two proteins with identical N-terminal, TCP and R domains but different C-terminal (C-t) domains of 36 and 57 amino acids respectively, due to a frameshift caused by the alternative splicing of intron I (Figure 1 b). For *SIBRC1b,* inventors isolated a single type of cDNA containing a 1089 bp long ORF split by one intron (Figure 1 b).

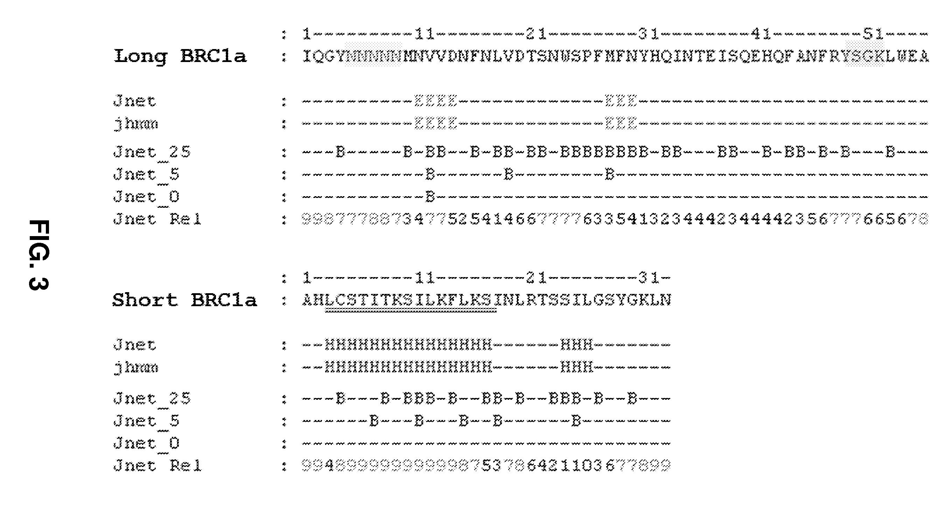

Alignment of the short and long cDNAs of *SIBRC1a,* the cDNA of *SIBRC1b* and the *SIBRC1a* and *SIBRC1b* genomic sequences confirmed that intron II of *SIBRC1a* was co-linear with the intron of *SIBRC1b,* suggesting that this intron was also spliced in the common ancestor, before duplication. In contrast, intron I of *SIBRC1a* could have evolved later, after duplication: it had frame conservation and a high sequence similarity to a coding (unspliced) region of *SIBRC1b.* Moreover, the C-t peptide encoded by the long *SIBRC1a* cDNA (which retained intron I) had sequence similarity to the C-t peptide of SIBRC1b, supporting that this frame was translated in the common ancestor, which did not splice this region. The putative splicing sites (gt/ag) of intron I were found in all the *Solanum BRC1a* genes studied but not in *BRC1b* genes, suggesting that they evolved within the *BRC1a* clade. Interestingly, the C-t region of the short BRC1 a protein was predicted to form a strongly amphpathic a-helix (Figure 3) highly conserved in all the *Solanum* species analyzed. On the other hand the C-t peptide of the long SIBRC1a protein was predicted to have an extended secondary structure (Figure 3) like that of SIBRC1b. These results indicate that, after the separation of Arabidopsis and Solanaceae, a duplication of an ancestral *BRC1*-like gene, which had one intron, generated two gene copies, *BRC1a* and *BRC1b.* A new alternative splicing site evolved in the *BRC1a* clade generating a divergent transcript, with a 3'-terminal frameshift, encoding a protein with a novel C-t motif predicted to form a strongly amphipathic α-helix.

Since the putative splicing sites (gt/ag) of intron I were found in all the *Solanum BRC1a* genes studied but not in *BRC1b* genes, inventors followed this pattern of alternative splicing of *BRC1a* gene in the genome sequence of *SpBRC1a* gene and, after was identified said intron I, inventors predicted the short cDNA coding for SEQ ID NO: 1 and the long cDNA coding for SEQ ID NO: 2.

### EXAMPLE 2. SpBRC1a acts as a suppressor of shoot branching in tomato Role of SIBRC1a and SIBRC1b.

The finding of two tomato *BRC1*-like paralogs more closely-related to each other than with *AtBRC1,* suggests that a duplication of *BRC1* took place after the separation of the Brassicaceae and Solanaceae. These two *BRC1*-like genes map to different chromosomes, suggesting that they did not originate by segmental duplication but rather by chromosomal duplication or during the whole-genome duplication (WGD) occurred in Solanaceae 60-70 mya. Duplication, a common event in the evolution of genomes, is usually followed by loss of one of the gene copies by accumulation of deleterious mutations. In Arabidopsis, for instance, a single *BRC1* gene has been found, although this species has undergone several WGD suggesting that all the other *BRC1*-like gene copies have been lost. A large number of models have been proposed to explain fixation, maintenance and evolution of duplicate genes.

These results indicate that in the case of tomato *BRC1*-like genes, *SIBRC1b* seems to have retained the ancestral *BRC1*-like gene function in the suppression of shoot branching while inventors have not been able to establish any relevant role in this process for *SIBRC1a.* Consistently, an asymmetrical distribution of selective constraints has been detected in the evolution of *Solanum BRC1a* and *BRC1b* lineages: the coding sequence of *BRC1b* genes have evolved under a strong purifying selection while the *BRC1a* lineage had experienced a decrease of evolutionary constraints.

### 2.1. Role of SIBRC1a and SIBRC1b in tomato and its evolution in Solanum genus.

As far as it is known, *SIBRC1b* acts as a suppressor of shoot branching in tomato whereas *SIBRC1a* does not play major role in the control of shoot branching in tomato (WO 2010/081917 A1). In the above-mentioned patent application inventors arrived to this conclusion after carrying out experiments using RNAi for each of the genes *SIBRC1a* and *SIBRC1b,* taking this fact into account in the final claims, because neither the use of *SIBRC1a* nor the use of *SIBRC1a*-RNAi contributed to solve any technical problem. In the present application inventors repeated the experiments of silencing and the same results that it is shown in WO 2010/081917 were obtained; *SIBRC1b* loss-of function leads to increased branch outgrowth in tomato but *SIBRC1a* loss-of function does not cause bud outgrowth. As a result, these examples are excluded in the present application because they do not provide more relevant information.

### 2.1.1. Role of SIBRC1a

The *Solanum lycopersicum* wild-type branching patterns of strong *Slbrc1a*-v220i mutants could indicate that *SIBRC1a* does not play a major role in the suppression of axillary bud outgrowth in tomato, raising the possibility that this gene has become non-functional in this species. Other signs of pseudogenization such as the accumulation of missense mutations have not been found in this tomato gene. Moreover, transcription is tightly regulated spatially and temporally, and alternative splicing takes place, indicating that transcriptional and post-transcriptional regulation have not been lost. No signs of a trend towards pseudogenization have been detected in the evolution of the *Solanum BRC1a* lineage, either. These results indicate that the fast evolution rate found in this clade is not due to relaxation (usually associated with loss of function), but rather to positive selection and adaptation. Moreover, inventors have identified several protein sites, targets of positive selection and evolving at high evolution rate, located in domains potentially involved in stability (PEST domain) and transcriptional activity, aspects which could greatly affect protein function. In addition, the alternative splicing evolved within the *BRC1a* clade gives rise to a novel transcript (identified both in tomato and potato) encoding a divergent protein with a C-t amphipathic helix, secondary structure often involved in protein-protein interactions, dimerization and transcriptional activation or repression. Inventors speculate that this novel domain, generated by a 3' terminal (3'-t) frameshift of the *BRC1a* transcripts, could also be contributing to the structural (and perhaps functional) divergence of BRC1 a proteins. 3'-t frameshift mutations have been proposed to be responsible for the evolution of novel protein domains, instrumental to the diversification of transcription factor families. In summary, the *BRC1a* clade could be diversifying rather than leading to loss of function and pseudogenization.

In the particular case of the tomato *SIBRC1a* gene, its low transcription levels and the apparently irrelevant function could be result of the recent artificial evolution associated to tomato domestication. Under-expressor alleles could have been fixed during the selection of other linked loci controlling selected traits. Alternatively, *SIBRC1a* could be functional but only up-regulated under certain developmental or environmental conditions not yet identified. So far, inventors have not detected up-regulation of *SIBRC1a* in plants with reduced *SIBRC1b* function, for instance, but other genetic or environmental situations might lead to transcriptional activation of *SIBRC1a.*

### 2.1.2. Role of SIBRC1b

The excess of shoot branching displayed by *SIBRC1b* loss-of-function plants resembles that of *tb1*/*brc1* mutants in other species (Aguilar-Martinez et al. 2007. Plant Cell, 19: 458-472; Doebley et al. 1997. Nature, 386: 485-488; Finlayson 2007. Plant Cell Physiol., 48: 667-677; Minakuchi et al. 2010. Plant Cell Physiol, 51: 1127-1135, Takeda et al. 2003. Plant J., 33: 513-520) and indicates that *SIBRC1b* must have retained the ancestral role in branch growth suppression. This is consistent with the low evolution rate (w) of the *BRC1b* genes in *Solanum. SIBRC1b* cis-regulatory regions and trans-acting factors also seem to be conserved: *SIBRC1b* is mainly expressed in arrested axillary buds, especially in basal nodes, like *AtBRC1* and rice *fine culm1,* and it responds to decapitation like *AtBRC1* (Aguilar-Martinez *et al.* 2007, Arite et al. 2007. Plant Journal, 51: 1019-1029). In Arabidopsis and rice it has been proposed that *AtBRC1* and *FC1,* respectively, are downstream of the strigolactone pathway (Aguilar-Martinez et al. 2007. Plant Cell, 19: 458-472; Minakuchi et al. 2010. Plant Cell Physiol, 51: 1127-1135). Although in tomato this needs to be tested, loss of function of *SICCD7,* a gene involved in tomato strigolactone synthesis (Vogel et al. 2009. Plant J, 61, 300-311; Koltai et al. 2010. J Exp Bot, 61: 1739-1749) causes alterations in vegetative but not in sympodial branching, similarly to loss of *SIBRC1b* function. However, *Slccd7* phenotypes are much stronger than those of *SIBRC1b* RNAi lines suggesting that additional factors may be controlled by this gene. Other related TCP genes such as *SIBRC1a, SIBRC2a* and *SIBRC2b,* are expressed in axillary buds at much lower levels than *SIBRC1b* but inventors cannot rule out that, in some conditions, they are relevant for this process.

### 2.2. Use of SpBRC1a as a suppressor of axillary shoot branching in tomato

Different tomato seed stocks obtained from the Tomato Genetics Resource Centre (TGRC) at the University of California Davis were grown. Special mention can be done with respect to the introgression line IL3-5. As it is indicated in the "chromosomal mapping" section, mapping was done in a *Solanum pennellii* introgression population using primers combinations and, after detection of the corresponding PCR fragments, the inventors concluded that *BRC1a* mapped to IL3-5 (Figure 2), in addition *BRC1b* mapped to IL6-2 and *BRC2a* to IL4-1. Therefore, the introgressed fragment from *Solanum pennellii* contains *SpBRC1a* gene. For these plants IL3-5, neither "branchless" phenotype nor the expression of *SpBRC1a* has been mentioned to date.

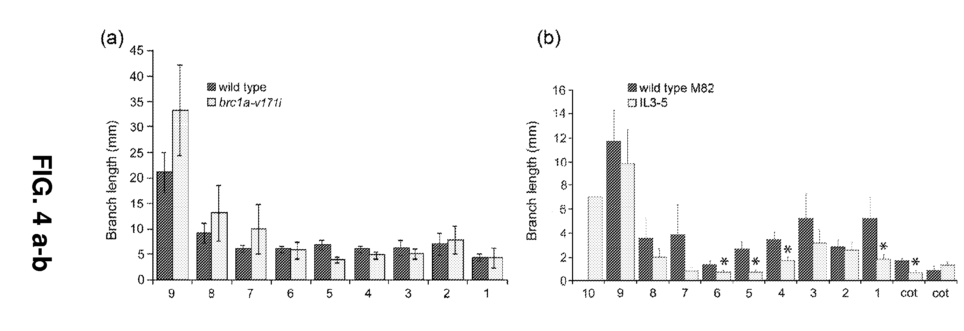

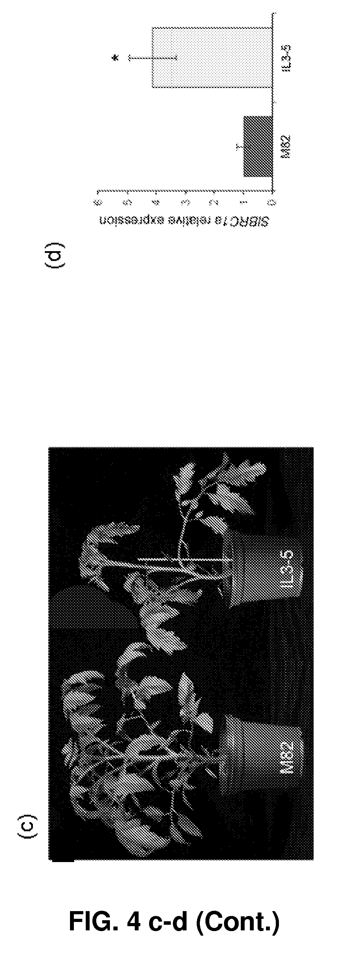

Interestingly, as shown in figure 4, IL3-5 lines, carrying a genomic region comprising a *SpBRC1a* gene, expressed this gene, *SpBRC1a,* at four times higher levels than *SIBRC1a* is expressed in M82 control plants and they display stronger apical dominance than M82 plants. These results demonstrate that introgression of expressed wild *SpBRC1a* genes into production tomato lines generates plants with improved architecture and reduced lateral shoot branching (figures 4 and 5).

### EXAMPLE 3. SpBRC1a is the responsible for the strong apical dominance of introgression line IL3-5 plants and also of SpBRC1a Solanum lycopersicum transgenic plants.

To confirm that *SpBRC1a,* contained in the introgression line IL3-5, is the gene responsible for the strong apical dominance of these lines, inventors caused silencing of *SpBRC1a* by generating RNAi transgenic lines carrying a sequence that is identical to part of its coding sequence. Inventors compared the branching phenotype of transgenic plants with those of M82 and with those carrying an empty binary vector. *SpBRC1a* was the responsible for the branch suppression RNAi lines having a similar number of branches as M82 plants.

### 3.1. SpBRC1a and the strong apical dominance of introgression line IL3-5 plants.

### 3.1.1. CaMV35S:RNAi Constructs

A *BRC1a*-specific PCR product (225 bp) was cloned into the vector pHANNIBAL (CSIRO) using restriction sites BamHI/ClaI and XhoI/KpnI for the first and second cloning, respectively. Primers used were A and B:
A: 5'GGGGCTCGAGGGATCCTGGCTACTCACAAAGTCAAAATCAGCG 3' (SEQ ID NO: 17).
B: 5'GGGGGGTACCATCGATAACAACTGGATGAATTATTGCCCTACG 3' (SEQ ID NO: 18).

The pHANNIBAL cassettes were digested with NotI and subcloned in the NotI site of Bluescript SK+. The cassette was then digested with SacI/SmaI and cloned in the MCS of the binary vector pBIN19. For the controls inventors will use an empty pBIN19 vector.

### 3.1.2. Generation of IL3-5 Tomato Transgenic Plants comprising the RNAi Constructs.

Binary vectors were transformed into *Agrobacterium tumefaciens* strain LBA4404. To generate stable transgenic tomato plants inventors will use the *Solanum pennellii* introgression line IL3-5 in the background of S. *lycopersicum* cv. M-82 (LA4060). Inventors transformed cotyledons according to (Ellul et al. 2003. Theor Appl Genet, 106: 231-238). Shoots regenerated from calli grown in kanamycin were transferred to non-selective root inducing media and were confirmed for transgene integration by PCR using primers Agri51 and PDK_Hannibal_51.
Agri51: 5'CAACCACGTCTTCAAAGCAA 3' (SEQ ID NO: 19).
PDK_Hannibal_51: 5'ATCTTCTTCGTCTTACACATCACTTG 3' (SEQ ID NO: 20).

Levels of transgene expression were quantified by Real-Time Q-PCR in RNA from leaves. Ploidy level of transformants was analyzed in leaf tissue, using wild type tissue as diploid reference. Nuclei were isolated according to (Galbraith et al. 1983. Science, 220: 1049-1051). Cytometry analyses were performed using a flow cytometer Beckman Coulter EPICS XL-MCL. Branch lengths were measured in mm immediately after anthesis of the first flower

### 3.2. SpBRC1a Solanum lycopersicum transgenic plants show branchless phenotype.

In addition, in order to confirm that *SpBRC1a* contributes to promote a stronger apical dominance than that of control plants, inventors generated transgenic lines carrying the genomic sequence of *SpBRC1a*; SEQ ID NO: 5. Inventors compared the branching phenotype of transgenic plants with those of M82 control plants and with those carrying an empty binary vector. Inventors observed that transgenic plants have fewer number of branches than M82 plants.

*SpBRC1a* was cloned in pBIN19 vector under the control of the *SpBRC1a* promoters. Binary vectors were transformed into *Agrobacterium tumefaciens* strain LBA4404 and transgenic plants of S. *lycopersicum* cv. M-82 were generated after regeneration.

### EXPERIMENTAL PROCEDURES

### Plant material

Tomato seed stocks were obtained from the Tomato Genetics Resource Centre (TGRC) at the University of California Davis or kindly provided by R. Fernández Muñoz (Estación Experimental La Mayora, Murcia, Spain). Accession numbers: Moneymaker (MM), LA2706; *Solanum pennellii,* LA0716; *Solanum pimpinellifolium,* PE-2; *Solanum habrochaites,* LA1777; *Solanum galapagense,* LA0317; *Solanum arcanum,* PE-30; *Solanum lycopersicum,* LA2706; *Solanum habrochaites glabratum,* PI 126449; *Solanum huaylasense,* LA2561; *Solanum chilense* (1969), LA1969; *Solanum peruvianum,* LA0098; *Solanum chmielewskii,* LA1028; *Solanum corneliomulleri,* LA0366; *Solanum pennellii* introgression lines in the background of *S. lycopersicum* cv. M-82, IL3-5, LA4047; LA4060. TILLING *Slbrc1a* point mutant alleles were obtained from tomato M82 EMS-mutagenized population and a Red Setter EMS-mutagenized population. Seeds were germinated in soil and transplanted to 1.5L pots and grown in chambers at 21ºC, long photoperiod (16 h light 8 hours dark), PAR 90 mmol m2 s-1.

### Identification and isolation of tomato BRC1-like genes

Inventors carried out BLAST searches at the TIGR Solanaceae Genomics Resource site (http://jcvi.org/potato/sol_ma_blast.shtml), TIGR Plant Transcript Assemblies Database (http://plantta.jcvi.org/) and SOL Genomics Network Tomato Gene Index website (http://solgenomics.net/) using the TCP domain of *AtBRC1.* To obtain full length cDNAs, RNA from dissected axillary buds was extracted with the guanidine-HCl method. cDNA was synthesized and full length cDNAs were isolated using primers indicated in Table S2.

### Chromosomal mapping of the CYC/TB1-like genes

Genomic fragments of *BRC1a, BRC1b* and *BRC2a* were amplified from *L. pennellii* and sequenced to find PCR based markers. Mapping was done in an *L. pennellii* introgression population (Eshed and Zamir 1995. Genetics, 141: 1147-1162) using primer combinations TCP1-F2 (5' CCTCATAAAAGGGAATCAAGGGA 3', SEQ ID NO: 13)/Le3 (5' ATTGAGAATGACTTGAAAGATAAAGATGAG 3'; SEQ ID NO: 14) and the restriction enzyme Csp6I to detect a CAPS for BRC1 a and TCP3-F2 (5' CCTCATAAAAGGGAATCAAGGGA 3'; SEQ ID NO: 15) /TCP3-R2 (5' TATTGAAAAATCGCGCACGTA 3'; SEQ ID NO: 16) and BspHI for BRC2a. A PCR-fragment length polymorphism for *BRC1b* was detected using primers TCP2-F2/LeTCP2 cDNA-R. *BRC1a* mapped to IL3-5, *BRC1b* to IL6-2 and *BRC2a* to IL4-1.

### Phenotypic analysis

Branch lengths were measured in mm immediately after anthesis of the first flower.

### Real-Time PCR

Plant tissue was harvested and RNA was isolated with the guanidine-HCl method. Each biological replicate came from individually dissected axillary buds (Ax buds) from nodes 1-2, 3-4, 5-6 and 7-8 from 12-15 individuals. For the other tissues (seed, stem, leaf, floral organs) each replicate contained material from 7-9 individuals. Traces of DNA were eliminated with TURBO DNA-free© (Ambion). Two □g of RNA were used to make cDNA with the High-Capacity cDNA Archive Kit (Applied Biosystems). Quantitative PCR was performed with SYBR® Green PCR Master Mix (Applied Biosystems) and the Applied Biosystems 7500 real-time PCR system, according to the manufacturer's instructions. Primers used are in Table S2. Three biological replicates were analyzed in each case. Ct values were obtained with the 7500 Systems SDS software 1.3.1.21 (Applied Biosystems). Relative fold expression changes were calculated as described in (Aguilar-Martinez et al. 2007. Plant Cell, 19: 458-472). ACTIN8 whose expression levels are constant in all the tissues and conditions analyzed was used as a normalizer. In all the figures the calibrator is the sample with the highest expression.

### Isolation of BRC1-like genes from wild tomato species

Young leaf tissue was collected in N(I), grinded and used to extract genomic DNA with the DNeasy Plant Mini kit (QIAGEN). To amplify the coding regions of *BRC1a* and *BRC1b* inventors carried out nested Pwo polymerase (ROCHE) PCR reactions according to manufacturer's instructions using primers indicated in Table S2.

### Phylogenetic analyses

TCP domain-coding DNA sequences were aligned with MUSCLE (v3.7) and default parameters (Edgar 2004. Nucleic Acids Res, 32: 1792-1797). Test of best nucleotide substitution evolutionary model was done with MODELTEST. The best fit model (AIC selection) was K81+G+I (w parameter for gamma distribution=0.725). Maximum likelihood (ML) tree reconstruction with the best model and 1000 bootstrap pseudoreplicates was run in PhyML (v3.0 aLRT). The complete phylogenetic pipeline was run in Phylemon web server. Tree was represented with TreeDyn (v198.3).

## Claims

1. An isolated polynucleotide, encoding a polypeptide comprising:
a. an amino acidic sequence from *Solanum pennellii* which is at least 95% identical over the full length of SEQ ID NO: 1 or of SEQ ID NO: 2, or
b. the amino acidic sequence from *Solanum pennellii* SEQ ID NO: 1 or SEQ ID NO: 2.

2. An isolated polynucleotide, encoding a polypeptide consisting of:
a. an amino acidic sequence from *Solanum pennellii* which is at least 95% identical over the full length of SEQ ID NO: 1 or of SEQ ID NO: 2, or
b. the amino acidic sequence from *Solanum pennellii* SEQ ID NO: 1 or SEQ ID NO: 2.

3. The polynucleotide according to claim 2, wherein said polynucleotide is
a. SEQ ID NO: 3 encoding SEQ ID NO: 1, or
b. SEQ ID NO: 4 encoding SEQ ID NO: 2.

4. The polynucleotide according to claim 2, wherein said polynucleotide is the genomic sequence SEQ ID NO: 5.

5. The polynucleotide according to any of claims 1 to 4 operatively linked to any regulatory sequence that controls the expression of said polynucleotide, preferably the regulatory sequence is SEQ ID NO: 6, SEQ ID NO: 7 or CaMV35S.

6. An expression product of the polynucleotide according to any of claims 1 to 5.

7. An expression vector comprising the polynucleotide according to any of claims 1 to 5.

8. A host cell having the expression vector comprising the polynucleotide according to any of claims 2 to 5 wherein if the host cell is a plant cell, said plant cell is not a *Solanum pennellii* cell.

9. A use of the expression vector according to claim 7, or the host cell according to claim 8, to produce a tomato plant or germplasm thereof but not a *Solanum pennellii* plant or germplasm thereof, having fewer and/or shorter axillary shoots than a control plant.

10. The use according to claim 9, wherein the produced tomato plant, or germplasm thereof, is classified as *Solanum lycopersicum* species.

11. A tomato plant, but not of the species *Solanum pennellii,* having the expression vector comprising the polynucleotide according to any of claims 2 to 5; or comprising the host cell according to claim 8, wherein said plant has fewer and/or shorter axillary shoots than a control plant.

12. The tomato plant according to claim 11, wherein said tomato plant is a *Solanum lycopersicum* plant.

13. The tomato plant according to any of claims 11 or 12, wherein the expression of the polynucleotide is greater than the expression of the homologous native gene *BRC1a.*

14. A germplasm of the tomato plant according to any of claims 11 to 13, preferably the germplasm is a seed or pollen.

15. A method for producing tomato plants having fewer and/or shorter axillary shoots than a control plant, comprising:
a. Transforming at least a tomato plant cell with the expression vector comprising the polynucleotide according to any of claims 2 to 5 and
b. growing the plant cell obtained in the step (a) in a suitable medium to produce at least a plant which expresses the heterologous polynucleotide.

16. The method according to claim 15, wherein the tomato plant is a *Solanum lycopersicum* plant.

## Patentansprüche

1. Isoliertes Polynukleotid, das ein Polypeptid codiert, umfassend:
a. eine Aminosäuresequenz aus *Solanum pennellii,* die über die gesamte Länge der SEQ ID NO: 1 oder der SEQ ID NO: 2 zu mindestens 95 % identisch ist, oder
b. die Aminosäuresequenz aus *Solanum pennellii* SEQ ID NO: 1 oder SEQ ID NO: 2.

2. Isoliertes Polynukleotid, das ein Polypeptid codiert, bestehend aus:
a. einer Aminosäuresequenz aus *Solanum pennellii,* die über die gesamte Länge der SEQ ID NO: 1 oder der SEQ ID NO: 2 zu mindestens 95 % identisch ist, oder
b. der Aminosäuresequenz aus *Solanum pennellii* SEQ ID NO: 1 oder SEQ ID NO: 2.

3. Polynukleotid nach Anspruch 2, wobei das besagte Polynukleotid
a. die für SEQ ID NO: 1 codierende SEQ ID NO: 3 oder
b. die für SEQ ID NO: 2 codierende SEQ ID NO: 4
ist.

4. Polynukleotid nach Anspruch 2, wobei das besagte Polynukleotid die genomische Sequenz SEQ ID NO: 5 ist.

5. Polynukleotid nach einem der Ansprüche 1 bis 4, das funktionell an eine regulatorische Sequenz gebunden ist, die die Expression des besagten Polynukleotids steuert, wobei die regulatorische Sequenz vorzugsweise SEQ ID NO: 6, SEQ ID NO: 7 oder CaMV35S ist.

6. Expressionsprodukt des Polynukleotids nach einem der Ansprüche 1 bis 5.

7. Expressionsvektor, der das Polynukleotid nach einem der Ansprüche 1 bis 5 umfasst.

8. Wirtszelle mit dem Expressionsvektor, der das Polynukleotid nach einem der Ansprüche 2 bis 5 umfasst, wobei wenn die Wirtszelle eine Pflanzenzelle ist, die besagte Pflanzenzelle keine Zelle von *Solanum pennellii* ist.

9. Verwendung des Expressionsvektors nach Anspruch 7 oder der Wirtszelle nach Anspruch 8 zur Herstellung einer Tomatenpflanze oder eines Keimplasmas davon, jedoch keine Pflanze der Art *Solanum pennellii* oder Keimplasma davon, die weniger und/oder kürzere Achselknospen aufweist als eine Kontrollpflanze.

10. Verwendung nach Anspruch 9, wobei die hergestellte Tomatenpflanze oder das Keimplasma davon als die Art *Solanum lycopersicum* eingestuft wird.

11. Tomatenpflanze, jedoch nicht der Art *Solanum pennellii,* die den Expressionsvektor aufweist, der das Polynukleotid nach einem der Ansprüche 2 bis 5 umfasst; oder die die Wirtszelle nach Anspruch 8 umfasst, wobei die besagte Pflanze weniger und/oder kürzere Achselknospen aufweist als eine Kontrollpflanze.

12. Tomatenpflanze nach Anspruch 11, wobei die besagte Tomatenpflanze eine Pflanze der Art *Solanum lycopersicum* ist.

13. Tomatenpflanze nach einem der Ansprüche 11 oder 12, worin die Expression des Polynukleotids größer ist als die Expression des homologen nativen Gens *BRC1a.*

14. Keimplasma der Tomatenpflanze nach einem der Ansprüche 11 bis 13, wobei das Keimplasma vorzugsweise Saatgut oder Pollen ist.

15. Verfahren zur Herstellung von Tomatenpflanzen, die weniger und/oder kürzere Achselknospen aufweisen als eine Kontrollpflanze, umfassend:
a. die Transformation mindestens einer Zelle der Tomatenpflanze mit dem Expressionsvektor, der das Polynukleotid nach einem der Ansprüche 2 bis 5 umfasst, und
b. das Züchten der in Schritt (a) erhaltenen Pflanzenzelle in einem geeigneten Medium, um mindestens eine Pflanze herzustellen, die das heterologe Polynukleotid exprimiert.

16. Verfahren nach Anspruch 15, wobei die Tomatenpflanze eine Pflanze der Art *Solanum lycopersicum* ist.

## Revendications

1. Polynucléotide isolé, codant un polypeptide et comprenant :
a. une séquence d'acides aminés de *Solanum pennellii* au moins identique à 95% sur toute la longueur de la SEQ ID N°: 1 ou SEQ ID N°: 2, ou
b. la séquence d'acides aminés de *Solanum pennellii* SEQ ID N°: 1 ou SEQ ID N°: 2.

2. Polynucléotide isolé, codant un polypeptide consistant en :
a. une séquence d'acides aminés de *Solanum pennellii* qui est au moins identique à 95% sur toute la longueur de la SEQ ID N°: 1 ou la SEQ ID N°: 2, ou
b. la séquence d'acides aminés de *Solanum pennellii* SEQ ID N°: 1 ou SEQ ID N°: 2.

3. Polynucléotide selon la revendication 2, où ledit polynucléotide est
a. la SEQ ID N°: 3 codant la SEQ ID N°: 1, ou
b. la SEQ ID N°: 4 codant la SEQ ID N°: 2.

4. Polynucléotide selon la revendication 2, où ledit polynucléotide est la séquence génomique SEQ ID N°: 5.

5. Polynucléotide selon l'une quelconque des revendications 1 à 4 lié de manière fonctionnelle à l'une des séquences régulatrices qui contrôle l'expression dudit polynucléotide, de préférence la séquence régulatrice est la SEQ ID N°: 6, la SEQ ID N°: 7 ou CaMV35S.

6. Produit d'expression du polynucléotide selon l'une quelconque des revendications 1 à 5.

7. Vecteur d'expression comprenant le polynucléotide selon l'une quelconque des revendications 1 à 5.

8. Cellule hôte ayant le vecteur d'expression comprenant le polynucléotide selon l'une quelconque des revendications 2 à 5, dans lequel, si la cellule hôte est une cellule végétale, ladite cellule végétale n'est pas une cellule de *Solanum pennellii.*

9. Utilisation du vecteur d'expression selon la revendication 7, ou de la cellule hôte selon la revendication 8, pour la production d'un plant de tomate ou de son germoplasme et non pas d'une plante de *Solanum pennellii* ou de son germoplasme, ayant moins et/ou des pousses axillaires plus courtes par rapport à une plante de contrôle.

10. Utilisation selon la revendication 9, où la plante de tomate produite, ou son germoplasme, est classée comme une espèce *Solanum lycopersicum.*

11. Plante de tomate, mais pas de l'espèce *Solanum pennellii,* ayant le vecteur d'expression comprenant le polynucléotide selon l'une quelconque des revendications 2 à 5 ; ou comprenant la cellule hôte selon la revendication 8, où ladite plante possède moins de et/ou des pousses axillaires plus courtes par rapport à une plante de contrôle.

12. Plante de tomate selon la revendication 11, où ladite plante de tomate est une plante de *Solanum lycopersicum.*

13. Plante de tomate selon l'une quelconque des revendications 11 ou 12, où l'expression du polynucléotide est supérieure à l'expression du gène natif homologue *BRC1a.*

14. Germoplasme de plante de tomate selon l'une quelconque des revendications 11 à 13, de préférence le germoplasme est une graine ou du pollen.

15. Méthode de production de plantes de tomate ayant moins et/ou des pousses axillaires plus courtes par rapport à une plante de contrôle, et comprenant :
a. La transformation d'au moins une cellule végétale de tomate avec le vecteur d'expression comprenant le polynucléotide selon l'une quelconque des revendications 2 à 5 et
b. la croissance de la cellule végétale obtenue pendant l'étape (a) dans un milieu approprié pour produire au moins une plante exprimant le polynucléotide hétérologue.

16. Méthode selon la revendication 15, où la plante de tomate est une plante de *Solanum lycopersicum.*


### SEQUENCE LISTING

<110> Consejo Superior de Investigaciones Científicas
<120> SpBRANCHED1a of Solanum pennellii and tomato plants with reduced branching comprising this heterologous SpBRANCHED1a gene
<130> EP2537.2
<160> 20
<170> PatentIn version 3.5
<210> 1
   <211> 313
   <212> PRT
   <213> Solanum pennellii
<400> 1
   Met Tyr Pro Ser Ser Asn Asn Ser Pro Asn Ile Ser Ser Ser Ser Ser
   1               5                   10                  15
   Phe Phe His Ile Asn Ile Pro Ser Pro Ser Met Gln Tyr Glu Pro Glu
               20                  25                  30
   Phe Ile Gln Tyr Phe Gln Asp Phe Gln Phe Ile Gln Pro Ser Tyr Asp
           35                  40                  45
   Gln Asn Asn Leu Asp Thr Asn Ile Thr Arg Glu Asp Ser Asp Lys Leu
       50                  55                  60
   Asp Lys Ile Glu Glu Asp Gln Ser Ile Ile Lys Ser Cys Asn Asn Lys
   65                  70                  75                  80
   Lys Asp Glu Lys Ser Ser Ser Ser Thr Ser Thr Ile Arg Arg Lys Asn
                   85                  90                  95
   Asn Lys Arg Thr Thr Ser Gly Ser Ala Gly Val Gly Pro Ser Lys Lys
               100                 105                 110
   Asp Arg His Ser Lys Ile Asn Thr Ala His Gly Pro Arg Asp Arg Arg
           115                 120                 125
   Met Arg Leu Ser Leu Glu Ile Ala Arg Lys Phe Phe Asn Leu Gln Asp
       130                 135                 140
   Leu Leu Gly Phe Asp Lys Ala Ser Lys Thr Val Glu Trp Leu Leu Thr
   145                 150                 155                 160
   Lys Ser Lys Ser Ala Val Asn Asp Leu Val Gln Lys Ile Asn Lys Asp
                   165                 170                 175
   Lys Cys Ser Gly Ser Thr Asn Pro Asn Ile Ala Thr Val Ser Ser Pro
               180                 185                 190
   Ser Glu Ser Cys Glu Val Ile Ser Gly Val Ile Asp Glu Ser Ala Ala
           195                 200                 205
   Thr Asn Thr Ala Glu Thr His Lys Gln Gln Lys Lys Lys Val Lys Ser
       210                 215                 220
   Ile Arg Arg Ala Ile Phe His Pro Val Val Ala Lys Glu Ser Arg Lys
   225                 230                 235                 240
   Glu Ala Arg Ala Arg Ala Arg Glu Arg Thr Ile Ile Lys Lys Ser Leu
                   245                 250                 255
   Asn Asp Asn Thr Asn Asn Asn Asn Asn Gly Asp Gln Ser Met Ala Asp
               260                 265                 270
   Glu Asp Leu Thr Arg Ser Leu Gly Ser Trp Asn Thr Thr Phe Glu Asp
           275                 280                 285
   His Gln Ser Ala Thr Gly Ala His Leu Cys Ser Thr Ile Thr Arg Ser
       290                 295                 300
   Ile Leu Lys Phe Leu Lys Ser Ile Asn
   305                 310
<210> 2
   <211> 349
   <212> PRT
   <213> Solanum pennellii
<400> 2
   Met Tyr Pro Ser Ser Asn Asn Ser Pro Asn Ile Ser Ser Ser Ser Ser
   1               5                   10                  15
   Phe Phe His Ile Asn Ile Pro Ser Pro Ser Met Gln Tyr Glu Pro Glu
               20                  25                  30
   Phe Ile Gln Tyr Phe Gln Asp Phe Gln Phe Ile Gln Pro Ser Tyr Asp
           35                  40                  45
   Gln Asn Asn Leu Asp Thr Asn Ile Thr Arg Glu Asp Ser Asp Lys Leu
       50                  55                  60
   Asp Lys Ile Glu Glu Asp Gln Ser Ile Ile Lys Ser Cys Asn Asn Lys
   65                  70                  75                  80
   Lys Asp Glu Lys Ser Ser Ser Ser Thr Ser Thr Ile Arg Arg Lys Asn
                   85                  90                  95
   Asn Lys Arg Thr Thr Ser Gly Ser Ala Gly Val Gly Pro Ser Lys Lys
               100                 105                 110
   Asp Arg His Ser Lys Ile Asn Thr Ala His Gly Pro Arg Asp Arg Arg
                   115                 120                 125
   Met Arg Leu Ser Leu Glu Ile Ala Arg Lys Phe Phe Asn Leu Gln Asp
       130                 135                 140
   Leu Leu Gly Phe Asp Lys Ala Ser Lys Thr Val Glu Trp Leu Leu Thr
   145                 150                 155                 160
   Lys Ser Lys Ser Ala Val Asn Asp Leu Val Gln Lys Ile Asn Lys Asp
                   165                 170                 175
   Lys Cys Ser Gly Ser Thr Asn Pro Asn Ile Ala Thr Val Ser Ser Pro
               180                 185                 190
   Ser Glu Ser Cys Glu Val Ile Ser Gly Val Ile Asp Glu Ser Ala Ala
           195                 200                 205
   Thr Asn Thr Ala Glu Thr His Lys Gln Gln Lys Lys Lys Val Lys Ser
       210                 215                 220
   Ile Arg Arg Ala Ile Phe His Pro Val Val Ala Lys Glu Ser Arg Lys
   225                 230                 235                 240
   Glu Ala Arg Ala Arg Ala Arg Glu Arg Thr Ile Ile Lys Lys Ser Leu
                   245                 250                 255
   Asn Asp Asn Thr Asn Asn Asn Asn Asn Gly Asp Gln Ser Met Ala Asp
               260                 265                 270
   Glu Asp Leu Thr Arg Ser Leu Gly Ser Trp Asn Thr Thr Phe Glu Asp
           275                 280                 285
   His Gln Ser Gly Ser Gln Gly Tyr Asn Asn Asn Asn Asn Asn Met Asn
       290                 295                 300
   Val Val Asp Asn Phe Asn Leu Val Asp Thr Ser Asn Trp Ser Pro Phe
   305                 310                 315                 320
   Met Phe Asn Tyr His Gln Ile Asn Thr Glu Ile Ser Gln Glu His Gln
                   325                 330                 335
   Leu Thr Asn Phe Gln Tyr Ser Gly Lys Leu Trp Glu Ala
               340                 345
<210> 3
   <211> 942
   <212> DNA
   <213> Solanum pennellii
<400> 3
   atgtaccctt cgagcaataa cagccccaat atttccagct cttcatcttt ttttcacatt        60
   aatattccat ctccttctat gcaatatgaa cccgaattca tccaatattt ccaagatttt       120
   caattcatcc aacctagtta cgatcagaat aatctcgata ccaatattac tagagaagat       180
   tcggacaaac tagataaaat agaagaagat caatcaatca taaaaagctg caataacaag       240
   aaggatgaga agagtagtag cagtactagt actattcgta gaaaaaacaa caagagaact       300
   acgagtggta gtgctggtgt aggaccttcg aagaaagata gacacagcaa aatcaacacg       360
   gcacatggcc caagagaccg aagaatgaga ctatcacttg aaattgctcg caaattcttc       420
   aatttgcaag acttgcttgg gttcgataaa gccagcaaaa ctgtagaatg gctactcaca       480
   aagtcaaaat cagcggtgaa cgatctggtt cagaaaatta acaaagacaa atgcagcggt       540
   agtacaaatc ctaatattgc tactgtatca tctccttccg aatcatgtga agttatctct       600
   ggagtaatcg acgaatcagc tgcaactaat acagcagaaa ctcacaagca acagaagaaa       660
   aaggttaagt cgattcgtag ggcaatattt catcccgttg ttgcaaagga atcaaggaaa       720
   gaagcaagag ctagggcaag ggaaagaaca ataataaaga aaagcctaaa tgataacacg       780
   aataataata ataatggtga tcaatctatg gctgatgagg atttaacaag atcattagga       840
   tcttggaata ctacatttga agatcatcaa tcagcaactg gagcccattt atgttcaact       900
   atcaccagat caatactgaa atttctcaag agcatcaatt ga                          942
<210> 4
   <211> 1050
   <212> DNA
   <213> Solanum pennellii
<400> 4 atgttcaact atcaccagat caatactgaa atttctcaag agcatcaatt gacgaacttc 1020
cagtattctg ggaagttatg ggaagcttaa 1050
<210> 5
   <211> 1296
   <212> DNA
   <213> Solanum pennellii
<400> 5
   atgtaccctt cgagcaataa cagccccaat atttccagct cttcatcttt ttttcacatt 60
   aatattccat ctccttctat gcaatatgaa cccgaattca tccaatattt ccaagatttt 120
   caattcatcc aacctagtta cgatcagaat aatctcgata ccaatattac tagagaagat 180
   tcggacaaac tagataaaat agaagaagat caatcaatca taaaaagctg caataacaag 240
   aaggatgaga agagtagtag cagtactagt actattcgta gaaaaaacaa caagagaact 300
   acgagtggta gtgctggtgt aggaccttcg aagaaagata gacacagcaa aatcaacacg 360
   gcacatggcc caagagaccg aagaatgaga ctatcacttg aaattgctcg caaattcttc 420
   aatttgcaag acttgcttgg gttcgataaa gccagcaaaa ctgtagaatg gctactcaca 480
   aagtcaaaat cagcggtgaa cgatctggtt cagaaaatta acaaagacaa atgcagcggt 540
   agtacaaatc ctaatattgc tactgtatca tctccttccg aatcatgtga agttatctct 600
   ggagtaatcg acgaatcagc tgcaactaat acagcagaaa ctcacaagca acagaagaaa 660
   aaggttaagt cgattcgtag ggcaatattt catcccgttg ttgcaaagga atcaaggaaa 720
   gaagcaagag ctagggcaag ggaaagaaca ataataaaga aaagcctaaa tgataacacg 780
   aataataata ataatggtga tcaatctatg gctgatgagg atttaacaag atcattagga 840
   tcttggaata ctacatttga agatcatcaa tcaggtagtc aaggctataa taataataat 900
   aataatatga atgttgttga taactttaat ttggtggata ctagcaactg gagcccattt 960
   atgttcaact atcaccagat caatactgaa atttctcaag aggtatgaat taatttaatt 1020
   agtgaattgt ttttttaatt attattaatc cgatcgcaaa gtatttattt gtattttttc 1080
   tgttgcagca tcaattgacg aacttccagt attctgggaa gttatgggaa gcttaattag 1140
   ggcaaggaag ttcttgttga tttagtgcaa cttcagaaga aggctatttg gtcatgttgg 1200
   taatgtaaaa tgaatttgtc tattttatta ttatgtgtat tccatgaaaa aacaacttgt 1260
   atttttctta taaataagct catctttatc tttcaa 1296
<210> 6
   <211> 1723
   <212> DNA
   <213> Solanum lycopersicum
<400> 6
   agtctgaacc cctttcacct caactgtggg aagcaggaag aattcaccaa aactttaata 60
   acatattcag taaaaatttt tataatgcgt ctaagaaaag taaaatgtac gtagaattta 120
   tcctgcctcg taaaaataaa gattgtatct aaaaaaaacc tcgactcaaa taatacatat 180
   taacaaaatt acaaattaac tatcactcaa ccccaatatt tacttcaagt tgttagggat      240
   cacttaaggg cctttctttt tctccttttt tttttttttt ttggagaaga tgaaggtgaa      300
   agagatggtg gatgatggag ctaggaaaga ggagattgaa gggtattttt tttgtcaaag      360
   tatgtgtcag ttgctatcac gtgaacttga aactaagggg caccattaga gaagacttta      420
   gctataatat acattcattt ctataaaaaa aaatcacmac ataaacatgc ccttttttaa      480
   cttagcttta atatatcttt taaatttgat tatgcagaaa tagatattta aatttatata      540
   aaatttaaaa agtctatcta acaatgttgt gtcctacata tattatatct ggtatgatat      600
   atatgtgtta cttgtttaat tttatataaa tttaaatatt tatttatgca aattcaaaat      660
   taagagatat aaatatcaag ctaaatcgaa gttcaatgaa atatatatat ataattatgc      720
   caatataaaa tcagtgtaac tatacaacaa gtactatagt gtcccctcca ctcttttttt      780
   ctcaaattcc ctttcatact ttaaactccc acatgagcta gctagagaag tctttttttt      840
   ttttaaagat tcgkggtgtt tacatcaatt taaacatatt ttgactaatt tcatagaata      900
   tttatcatct cttattaata acatgtgtca tattcataaa tgaatagaaa ttactaaata      960
   cagtagtact ycttttaatt tttttctaat aaaatttaaa cgtgaaacct catgattcct     1020
   aattatccac ttcagtaacc atcgactcac accaaccctt tggtgcaagc gaagccttct     1080
   ttatctttat agcagatagg ggtcctttga aaagatggaa gtacaattac acctctcttt     1140
   gtccctttgc aggtaataac ataacatgac ttttctttat cttcatcttt ctttctttgt     1200
   caacaagaac atacaccacc atgaatgtct ctcccattag ctaatatatt ccagctaact     1260
   agcttaaata tatagtgcta atacytgcac gaacacaaaa atagccacta atatacacct     1320
   atacctagct attattatta ttatcataat taagcacwca ccaagcaaca tacatgtaaa     1380
   gccacatatt tttaatcacc tgtctttctc aaccaaaaag ctatattatc atcattatat     1440
   tgaaaaaaaa attaaaaata accacatatc ctttcccact ttctctatgt gctatctttg     1500
   tattcaaaat ttatatatcc aagagaatta tgaagagtct ctctcaaaaa aagttttaat     1560
   taatttataa ccttttcttt tttcctactt tttgttgatg cagctaggta gctagattat     1620
   taaaagtgtc aaactgaaga agctgatgtt tgtggttatt tcaacttcaa tacaagtgtg     1680
   ctaggttgtc cttatcaacc agtttctttt ttttttttta aag                        1723
<210> 7
   <211> 665
   <212> DNA
   <213> Solanum lycopersicum
<400> 7
   tcacatgaag gggcacgata acaagttgtt cgtatccatc cattcacttc caacaatacc       60
   gctacgtacc actacctgct tccttcctat ccccagtctt tgtcaaactt cctttccctt      120
   tccaattact ttttctttaa tagagatgtt tgtttccttt tcccttcccc ccatattctt      180
   cccttttttt tttatctctc tttcacaata gtagcaccat gcctgtagct ttgatgctta      240
   gacgggcgca cgcgcacgcg cactcacaca actagaatag aatcactctc tctatatatt      300
   catagttatc aaaactactt atcatatacc aaaaaaaacc actgtcattc tcaagcaaat      360
   aatatttttt ttaaaaaaga agaactacat atatatatat agtactacta ctattttcat      420
   catcactttg gtcaatccat acagttctaa gtagtcattg cttcctctgt caaattactg      480
   tatacagtac attgaactag ctaggggaaa attaatctac taactctaat ttgtttgttt      540
   aattctcttc ttattgcagc tagatttgcc taattagcag aaaaaccaaa agctgtgttc      600
   atactgtctt tctcaagatc tagacccacc atatagaccg cctcaactac agctactcca      660
   caaga                                                                  665
<210> 8
   <211> 325
   <212> PRT
   <213> Solanum lycopersicum
<400> 8
   Met Tyr Pro Ser Ser Asn Tyr Ser Pro Asn Ile Ser Ser Ser Ser Ser
   1               5                   10                  15
   Phe Phe His Ile Asn Ile Pro Ser Pro Ser Met Gln Tyr Glu Pro Glu
               20                  25                  30
   Phe Ile Gln Tyr Phe His Asp Phe Gln Phe Ile Gln Pro Ser Tyr Asp
           35                  40                  45
   Gln Asn Thr Asn Ile Pro Ala Glu Glu Ala Ala Asp Ser Asp Lys Leu
       50                  55                  60
   Asp Lys Ile Glu Glu Asp Gln Ser Ile Ile Lys Ser Cys Asn Asn Asn
   65                  70                  75                  80
   Lys Lys Asp Glu Lys Ser Ser Ser Ser Thr Ser Thr Ile Arg Arg Lys
                   85                  90                  95
   Asn Asn Lys Arg Thr Thr Ser Gly Ser Ala Gly Val Gly Pro Ser Lys
               100                 105                 110
   Lys Asp Arg His Ser Lys Ile Asn Thr Ala His Gly Pro Arg Asp Arg
           115                 120                 125
   Arg Met Arg Leu Ser Leu Glu Ile Ala Arg Lys Phe Phe Asn Leu Gln
       130                 135                 140
   Asp Leu Leu Gly Phe Asp Lys Ala Ser Lys Thr Val Glu Trp Leu Leu
   145                 150                 155                 160
   Thr Lys Ser Lys Ser Ala Val Asn Asp Leu Val Gln Lys Ile Asn Lys
                   165                 170                 175
   Asp Lys Cys Ser Gly Ser Glu Asn Pro Asn Ile Ala Thr Val Ser Ser
   180 185 190
   Pro Ser Ala Glu Ser Cys Glu Val Ile Asp Glu Ser Ala Ala Thr Asn
   195 200 205
   Thr Ala Glu Thr Gln Lys Gln Gln Lys Lys Lys Val Lys Ser Ile Arg
   210 215 220
   Arg Ala Ile Ile His Pro Val Val Ala Lys Glu Ser Arg Lys Glu Ala
   225 230 235 240
   Arg Ala Arg Ala Arg Glu Arg Thr Ile Ile Lys Lys Ser Leu Asn Asp
   245 250 255
   Asn Thr Asn Asn Asn Asn Asn Gly Asp Gln Ser Met Ala Asp Glu Asp
   260 265 270
   Leu Thr Arg Ser Leu Arg Ser Trp Asn Thr Thr Phe Glu Asp His Gln
   275 280 285
   Ser Ala Ile Gly Ala His Leu Cys Ser Thr Ile Thr Lys Ser Ile Leu
   290 295 300
   Lys Phe Leu Lys Ser Ile Asn Leu Arg Thr Ser Ser Ile Leu Gly Ser
   305 310 315 320
   Tyr Gly Lys Leu Asn
   325
<210> 9
   <211> 300
   <212> PRT
   <213> Solanum tuberosum
<400> 9
   Met Tyr Pro Ser Ser Pro Asn Ile Ser Ser Ser Ser Ser Phe Phe His
   1               5                   10                  15
   Ile Asn Ile Pro Ser Pro Ser Met Gln Tyr Glu Pro Glu Phe Ile Gln
               20                  25                  30
   Tyr Phe His Asp Phe Gln Phe Ile Gln Pro Ala Ala Tyr Asp Gln Asn
           35                  40                  45
   Asn Leu Asp Thr Asn Ile Thr Ala Glu Glu Gly Asp His Lys Met Glu
       50                  55                  60
   Glu Asp Glu Leu Ile Met Lys Ser Cys Lys Asn Lys Lys Asp Glu Ser
   65                  70                  75                  80
   Thr Ser Thr Thr Thr Thr Ile Arg Arg Lys Asn Asn Lys Arg Thr Thr
                   85                  90                  95
   Ser Gly Thr Gly Val Gly Pro Ser Lys Lys Asp Arg His Ser Lys Ile
               100                 105                 110
   Asn Thr Ala His Gly Pro Arg Asp Arg Arg Met Arg Leu Ser Leu Glu
           115                 120                 125
   Ile Ala Arg Lys Phe Phe Asn Leu Gln Asp Leu Leu Gly Phe Asp Lys
       130                 135                 140
   Ala Ser Lys Thr Val Glu Trp Leu Leu Thr Lys Ser Lys Ser Ala Val
   145                 150                 155                 160
   Asn Asp Leu Val Gln Lys Ile Ser Lys Gly Lys Cys Ser Ala Ser Thr
                   165                 170                 175
   Asn Pro Asn Ile Gly Val Val Ser Ser Pro Ser Glu Ser Cys Glu Val
               180                 185                 190
   Ile Ser Gly Val Ile Asp Glu Ser Ala Ala Thr Asn Asn Thr His Lys
           195                 200                 205
   Gln Gln Lys Lys Lys Lys Ser Ile Arg Arg Ala Ile Phe His Pro Val
       210                 215                 220
   Val Ala Lys Glu Ser Arg Thr Glu Ala Arg Ala Arg Ala Arg Glu Arg
   225                 230                 235                 240
   Thr Lys Ile Lys Lys Ser Leu Asn Asn Asn Asn Gly Asp Gln Ser Met
                   245                 250                 255
   Ala Pro Asp Glu Asp Leu Thr Arg Ser Leu Gly Ser Trp Ser Thr Thr
               260                 265                 270
   Phe Glu Asp His Gln Ser Ala Ile Gly Ala His Leu Cys Ser Thr Ile
           275                 280                 285
   Thr Lys Ser Ile Leu Lys Phe Leu Arg Ser Ile Asn
       290                 295                 300
<210> 10
   <211> 346
   <212> PRT
   <213> Solanum lycopersicum
<400> 10
   Met Tyr Pro Ser Ser Asn Tyr Ser Pro Asn Ile Ser Ser Ser Ser Ser
   1               5                   10                  15
   Phe Phe His Ile Asn Ile Pro Ser Pro Ser Met Gln Tyr Glu Pro Glu
               20                  25                  30
   Phe Ile Gln Tyr Phe His Asp Phe Gln Phe Ile Gln Pro Ser Tyr Asp
           35                  40                  45
   Gln Asn Thr Asn Ile Pro Ala Glu Glu Ala Ala Asp Ser Asp Lys Leu
       50                  55                  60
   Asp Lys Ile Glu Glu Asp Gln Ser Ile Ile Lys Ser Cys Asn Asn Asn
   65                  70                  75                  80
   Lys Lys Asp Glu Lys Ser Ser Ser Ser Thr Ser Thr Ile Arg Arg Lys
                   85                  90                  95
   Asn Asn Lys Arg Thr Thr Ser Gly Ser Ala Gly Val Gly Pro Ser Lys
               100                 105                 110
   Lys Asp Arg His Ser Lys Ile Asn Thr Ala His Gly Pro Arg Asp Arg
           115                 120                 125
   Arg Met Arg Leu Ser Leu Glu Ile Ala Arg Lys Phe Phe Asn Leu Gln
       130                 135                 140
   Asp Leu Leu Gly Phe Asp Lys Ala Ser Lys Thr Val Glu Trp Leu Leu
   145                 150                 155                 160
   Thr Lys Ser Lys Ser Ala Val Asn Asp Leu Val Gln Lys Ile Asn Lys
                   165                 170                 175
   Asp Lys Cys Ser Gly Ser Glu Asn Pro Asn Ile Ala Thr Val Ser Ser
               180                 185                 190
   Pro Ser Ala Glu Ser Cys Glu Val Ile Asp Glu Ser Ala Ala Thr Asn
           195                 200                 205
   Thr Ala Glu Thr Gln Lys Gln Gln Lys Lys Lys Val Lys Ser Ile Arg
       210                 215                 220
   Arg Ala Ile Ile His Pro Val Val Ala Lys Glu Ser Arg Lys Glu Ala
   225                 230                 235                 240
   Arg Ala Arg Ala Arg Glu Arg Thr Ile Ile Lys Lys Ser Leu Asn Asp
                   245                 250                 255
   Asn Thr Asn Asn Asn Asn Asn Gly Asp Gln Ser Met Ala Asp Glu Asp
               260                 265                 270
   Leu Thr Arg Ser Leu Arg Ser Trp Asn Thr Thr Phe Glu Asp His Gln
           275                 280                 285
   Ser Gly Ile Gln Gly Tyr Asn Asn Asn Asn Asn Met Asn Val Val Asp
       290                 295                 300
   Asn Phe Asn Leu Val Asp Thr Ser Asn Trp Ser Pro Phe Met Phe Asn
   305                 310                 315                 320
   Tyr His Gln Ile Asn Thr Glu Ile Ser Gln Glu His Gln Phe Ala Asn
                   325                 330                 335
   Phe Gln Tyr Ser Gly Lys Leu Trp Glu Ala
               340                 345
<210> 11
   <211> 336
   <212> PRT
   <213> Solanum tuberosum
<400> 11
   Met Tyr Pro Ser Ser Pro Asn Ile Ser Ser Ser Ser Ser Phe Phe His
   1               5                   10                  15
   Ile Asn Ile Pro Ser Pro Ser Met Gln Tyr Glu Pro Glu Phe Ile Gln
               20                  25                  30
   Tyr Phe His Asp Phe Gln Phe Ile Gln Pro Ala Ala Tyr Asp Gln Asn
           35                  40                  45
   Asn Leu Asp Thr Asn Ile Thr Ala Glu Glu Gly Asp His Lys Met Glu
       50                  55                  60
   Glu Asp Glu Leu Ile Met Lys Ser Cys Lys Asn Lys Lys Asp Glu Ser
   65                  70                  75                  80
   Thr Ser Thr Thr Thr Thr Ile Arg Arg Lys Asn Asn Lys Arg Thr Thr
                   85                  90                  95
   Ser Gly Thr Gly Val Gly Pro Ser Lys Lys Asp Arg His Ser Lys Ile
               100                 105                 110
   Asn Thr Ala His Gly Pro Arg Asp Arg Arg Met Arg Leu Ser Leu Glu
           115                 120                 125
   Ile Ala Arg Lys Phe Phe Asn Leu Gln Asp Leu Leu Gly Phe Asp Lys
       130                 135                 140
   Ala Ser Lys Thr Val Glu Trp Leu Leu Thr Lys Ser Lys Ser Ala Val
   145                 150                 155                 160
   Asn Asp Leu Val Gln Lys Ile Ser Lys Gly Lys Cys Ser Ala Ser Thr
                   165                 170                 175
   Asn Pro Asn Ile Gly Val Val Ser Ser Pro Ser Glu Ser Cys Glu Val
               180                 185                 190
   Ile Ser Gly Val Ile Asp Glu Ser Ala Ala Thr Asn Asn Thr His Lys
           195                 200                 205
   Gln Gln Lys Lys Lys Lys Ser Ile Arg Arg Ala Ile Phe His Pro Val
       210                 215                 220
   Val Ala Lys Glu Ser Arg Thr Glu Ala Arg Ala Arg Ala Arg Glu Arg
   225                 230                 235                 240
   Thr Lys Ile Lys Lys Ser Leu Asn Asn Asn Asn Gly Asp Gln Ser Met
                   245                 250                 255
   Ala Pro Asp Glu Asp Leu Thr Arg Ser Leu Gly Ser Trp Ser Thr Thr
               260                 265                 270
   Phe Glu Asp His Gln Ser Gly Ile Gln Ala Tyr Asn Asn Thr Asn Asn
           275                 280                 285
   Ile Met Asn Ala Val Asp Asn Phe Asn Leu Val Asp Thr Ser Asn Trp
       290                 295                 300
   Ser Pro Phe Met Phe Asn Tyr His Gln Ile Asn Thr Glu Ile Ser Gln
   305                 310                 315                 320
   Glu His Gln Leu Thr Asn Phe Gln Tyr Pro Gly Lys Leu Trp Glu Ala
                   325                 330                 335
<210> 12
   <211> 362
   <212> PRT
   <213> Solanum pennellii
<400> 12
   Met Tyr Pro Pro Ser Asn Asn Asn Cys Asn Tyr Ser Pro Ile Leu Ser
   1 5 10 15
   Ser Leu Ile Cys Gln Asn Ile Pro Ser Ser Pro Cys Met Gln Tyr Glu
   20 25 30
   His Glu Leu Tyr Phe Gln Ser Phe Asn His Asp Asp Gln Tyr Tyr Phe
   35 40 45
   Gln Gln Gln Gln Leu Val Pro Leu Ile Asp Asp Leu Ser Pro His Ile
   50 55 60
   Leu Ala Asp Ser Cys Ile Glu Thr Ile Thr Lys Pro Ser Asn Cys Asn
   65 70 75 80
   His Val Leu Glu Gly Met Glu Glu Gly Arg Gly Glu Lys Lys Gly Asp
   85 90 95
   Asp Asp Val Arg Ser Ser Arg Ile Ser Ile Ser Lys Asn Asn Lys Arg
               100                 105                 110
   Ser Ser Lys Lys Asp Arg His Ser Lys Ile Asn Thr Ala Arg Gly Pro
           115                 120                 125
   Arg Asp Arg Arg Met Arg Leu Ser Leu Asp Ala Ala Arg Lys Phe Phe
       130                 135                 140
   Arg Leu Gln Asp Leu Leu Gly Phe Asp Lys Ala Ser Lys Thr Val Glu
   145                 150                 155                 160
   Trp Leu Leu Thr Gln Ser Asp Ser Ala Ile Glu Glu Leu Val Ala Ala
                   165                 170                 175
   Lys Gly Asn Asp Ala Gln Val Ala Gln Gln Thr Ser Cys Asn Thr Pro
               180                 185                 190
   Thr Thr Thr Thr Gly Ile Gly Ala Ile Cys Ala Ser Asn Ser Ile Ser
           195                 200                 205
   Glu Ser Cys Glu Val Ile Ser Gly Thr Asp Glu Thr Ser Ser Asn Asp
       210                 215                 220
   Lys Asn Lys Glu Thr Ala Gln Asp Glu Lys Lys Lys Arg Lys Lys Val
   225                 230                 235                 240
   Val Asn Ala Ala Arg Arg Ala Val Leu Glu Pro Leu Thr Lys Glu Ser
                   245                 250                 255
   Arg Asn Gln Ala Arg Ala Arg Ala Arg Glu Arg Thr Lys Ser Lys Lys
               260                 265                 270
   Met Ser Gln Thr Gly Lys Ser Lys Ala Pro Ala Asn Asp Leu Asn Pro
           275                 280                 285
   Ser Gly Ser Arg Arg Pro Ala Asn Lys Thr Cys Glu Glu Ala Gly Thr
       290                 295                 300
   His Glu Glu Leu Asn Phe His Gln Glu Lys Asn Ser Val Asp Asp Cys
   305                 310                 315                 320
   Asn Phe Met Val Asn Gly Asn Trp Asn Pro Phe Thr Ile Phe Ser Tyr
                   325                 330                 335
   His Glu Gln Tyr Ala Gly Ile Ser Asn Glu His Gln Leu Val Thr Asp
               340                 345                 350
   Leu Gln Phe Cys Gly Lys Leu Trp Glu Gly
           355                 360
<210> 13
   <211> 23
   <212> DNA
   <213> Artificial Sequence
<220>
   <223> TCP3-F2 forward primer of SlBRC2a from Solanum lycopersicum
<400> 13
   cctcataaaa gggaatcaag gga 23
<210> 14
   <211> 30
   <212> DNA
   <213> Artificial Sequence
<220>
   <223> Le3 reverse primer of SlBRC1a from Solanum lycopersicum
<400> 14
   attgagaatg acttgaaaga taaagatgag 30
<210> 15
   <211> 23
   <212> DNA
   <213> Artificial Sequence
<220>
   <223> TCP3-F2 forward primer of SlBRC2a from Solanum lycopersicum
<400> 15
   cctcataaaa gggaatcaag gga 23
<210> 16
   <211> 21
   <212> DNA
   <213> Artificial Sequence
<220>
   <223> TCP3-R2 reverse primer of SlBRC2a from Solanum lycopersicum
<400> 16
   tattgaaaaa tcgcgcacgt a 21
<210> 17
   <211> 43
   <212> DNA
   <213> Artificial Sequence
<220>
   <223> A forward primer
<400> 17
   ggggctcgag ggatcctggc tactcacaaa gtcaaaatca gcg 43
<210> 18
   <211> 43
   <212> DNA
   <213> Artificial Sequence
<220>
   <223> B reverse primer
<400> 18
   ggggggtacc atcgataaca actggatgaa ttattgccct acg 43
<210> 19
   <211> 20
   <212> DNA
   <213> Artificial Sequence
<220>
   <223> Agri51 forward primer
<400> 19
   caaccacgtc ttcaaagcaa 20
<210> 20
   <211> 26
   <212> DNA
   <213> Artificial Sequence
<220>
   <223> PDK_Hannibal_51 reverse primer
<400> 20
   atcttcttcg tcttacacat cacttg 26